(19) **Europäisches Patentamt / European Patent Office / Office européen des brevets**

(11) **EP 2 732 290 B1**

(12) **EUROPEAN PATENT SPECIFICATION**

(45) Date of publication and mention of the grant of the patent:
**25.05.2016 Bulletin 2016/21**

(51) Int Cl.:
*C12Q 1/68* (2006.01)   *G01N 33/68* (2006.01)

(21) Application number: **12738590.4**

(22) Date of filing: **16.07.2012**

(86) International application number:
**PCT/GB2012/051693**

(87) International publication number:
**WO 2013/011294 (24.01.2013 Gazette 2013/04)**

(54) **DIAGNOSIS OF ALZHEIMER'S DISEASE**

DIAGNOSE VON MORBUS ALZHEIMER

DIAGNOSTIC DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2011 GB 201112246**

(43) Date of publication of application:
**21.05.2014 Bulletin 2014/21**

(73) Proprietor: **The University of Birmingham Edgbaston Birmingham West Midlands B15 2TT (GB)**

(72) Inventor: **NAGY, Zsuzsanna Birmingham West Midlands B17 8EB (GB)**

(74) Representative: **Boult Wade Tennant Verulam Gardens 70 Gray's Inn Road London WC1X 8BT (GB)**

(56) References cited:
**WO-A1-03/007925      WO-A1-2011/007155
WO-A2-02/073212      WO-A2-2009/105549**

- ZHOU X ET AL: "P53-mediated G1/S checkpoint dysfunction in lymphocytes from Alzheimer's disease patients", NEUROSCIENCE LETTERS, LIMERICK, IE, vol. 468, no. 3, 14 January 2010 (2010-01-14), pages 320-325, XP026819934, ISSN: 0304-3940 [retrieved on 2009-11-13]
- BARTOLOME FERNANDO ET AL: "Distinct Regulation of Cell Cycle and Survival in Lymphocytes from Patients with Alzheimer's Disease and Amyotrophic Lateral Sclerosis", INTERNATIONAL JOURNAL OF CLINICAL AND EXPERIMENTAL PATHOLOGY, E-CENTURY PUBLISHING CORPORATION, US , vol. 2, no. 4 1 January 2009 (2009-01-01), pages 390-398, XP008156971, ISSN: 1936-2625 Retrieved from the Internet: URL:www.ijcep.com/IJCEP811004 [retrieved on 2008-12-01]
- KRISTOF ARNOLD S ET AL: "LY303511 (2-piperazinyl-8-phenyl-4H-1-benzopyran-4-one) acts via phosphatidylinositol 3-kinase-independent pathways to inhibit cell proliferation via mammalian target of rapamycin (mTOR)- and non- mTOR-dependent mechanisms", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 314, no. 3, 1 September 2005 (2005-09-01), pages 1134-1143, XP002443151, ISSN: 0022-3565, DOI: 10.1124/JPET.105.083550

• NAGY Z ET AL: "CELL CYCLE KINESIS IN LYMPHOCYTES IN THE DIAGNOSIS OF ALZHEIMER'S DISEASE", NEUROSCIENCE LETTERS, LIMERICK, IE, vol. 317, no. 2, 11 January 2002 (2002-01-11), pages 81-84, XP001106660, ISSN: 0304-3940, DOI: 10.1016/S0304-3940(01)02442-9

**Description**

**FIELD OF THE INVENTION**

The present invention relates to diagnosis and monitoring of Alzheimer's disease in the live subject. Particularly, although not exclusively, the invention relates to methods involving the measurement of differential gene expression in non-neuronal cells taken from human subjects suspected of having Alzheimer's disease. The invention also relates to a method by which to monitor mTOR signalling in a human cell as claimed.

**BACKGROUND TO THE INVENTION**

Alzheimer's disease is the most common form of dementia in older people. As a result of population aging worldwide, the prevalence of this disease is set to increase significantly in coming years. As such, there is an urgent need to develop better diagnostic tools and new treatments for people identified as having this disease.

Alzheimer's disease is a chronic neurodegenerative disorder characterised by selective loss of cortical neurons within the hippocampus and the temporal and frontal lobes of the brain. The pathological hallmarks of this disease consist of amyloid-$\beta$ plaques, which accumulate in the brain, and neurofibrillary tangles consisting of hyperphosphorylated tau protein present in affected neurons. The neurodegenerative process occurring in Alzheimer's disease is accompanied by progressive cognitive impairment leading ultimately to dementia in affected individuals.

There is currently no accepted "gold standard" diagnostic test for Alzheimer's disease in the live patient. This reflects the difficulties associated with identifying patients who would go on to be classified as having this disease at post mortem examination. Clinical diagnosis of Alzheimer's disease is typically based on evaluation of clinical criteria, such as the NINCDS/ADRDA criteria (McKhann, G. et al., (1984) Neurology 34: 939-944). These criteria are highly sensitive but have a low specificity. In particular, a significant proportion of patients who do not fulfil these criteria are found to have Alzheimer's disease at post-mortem examination.

The problem with the clinical diagnostic criteria used to date lies in the fact that patients are typically diagnosed once dementia has started to develop. However, at this stage of the disease, it is unlikely that any treatment will ever be able to reverse the damage caused by the neurodegenerative process. In order to improve the management of patients with Alzheimer's disease, methods and tools are needed which will allow for diagnosis at a much earlier stage of disease, ideally before cognitive symptoms are even present.

In this regard, there is now good evidence to suggest that the neuropathology underlying Alzheimer's disease begins years, maybe even a decade, prior to the diagnosis of clinical dementia (Forlenza et al., (2010) BMC Medicine 8:89). Based on these observations, the continuum of Alzheimer's disease progression has been classified into three phases:

    (i) asymptomatic Alzheimer's disease (preclinical stage);
    (ii) mild cognitive impairment (MCI) due to Alzheimer's disease (pre-dementia stage); and
    (iii) clinically-defined Alzheimer's disease (dementia).

Diagnostic tools and techniques that allow for identification of individuals with asymptomatic disease will be invaluable in improving the management of Alzheimer's disease. Moreover, it would be highly beneficial to be able to identify patients having MCI that will go to develop clinically-defined Alzheimer's disease. The current diagnostic criteria used to identify MCI have relatively poor prognostic value when it comes to identifying those at risk of progressing to Alzheimer's disease-associated dementia.

Steps have already been taken to revise the diagnostic criteria for Alzheimer's disease to tare account of the need to diagnosis individuals with this disease at a much earlier stage, preferably at the preclinical stage (Sperling et al., (2010) Criteria for preclinical Alzheimer's disease [www.alz.org/research/diagnostic_criteria/preclinical_recommendations.pdf]). In this regard, research is actively being carried out looking at the use of biomarkers to detect disease at an early stage (Gustaw-Rothenberg et al., (2010) Biomark. Med. 4(1):15-26).

To date, researchers have focussed on the measurement of biomarkers in humoral fluids, mainly cerebrospinal fluids, and biomarkers that may be detected using advanced neuroimaging methods. However, these approaches are not particularly suitable for convenient, wide-scale diagnostic testing.

In terms of the molecular changes underlying disease progression, it has been shown that Alzheimer's disease is associated with aberrant re-entry of neurons into the cell division cycle. This change appears to be an early event in disease pathogenesis preceding formation of both amyloid-$\beta$ plaques and neurofibrillary tangles. Taken together, these observations have led researchers to hypothesise that aberrant cell cycle regulation is a cause rather than a consequence of Alzheimer's disease (Nagy, (2005) J. Cell. Mol. Med. Vol.9,No.3: 531-541). Moreover, it is thought that detection of these cell cycle changes may be used to assist with diagnosis of Alzheimer's disease at an early stage, even in asymptomatic individuals.

In this regard, it has been observed that it is not cell cycle re-entry *per se* that contributes to Alzheimer's disease but rather the inability of neurons from Alzheimer's disease patients to respond appropriately to this cell-cycle re-entry. In particular, neurons from Alzheimer's disease patients are unable to initiate G1 arrest and subsequently undergo re-differentiation, as a result of a defect in the G1/S regulatory checkpoint.

Furthermore, this regulatory defect at the G1/S transition has been found to occur in cells other than neurons in individuals with Alzheimer's disease, for example lymphocytes. International application, WO02/073212, describes a method for diagnosis of Alzheimer's disease based around screening for the presence of a cell cycle regulatory defect at the G1/S phase checkpoint in non-neuronal cells, such as lymphocyte cultures isolated from individuals suspected of having disease. In particular, the differential sensitivity of the lymphocytes to a G1 inhibitor is used.to detect the defect in cell cycle control in cells from Alzheimer's disease patients.

WO2011/007155 already discloses methods assisting in the treatment of Alzheimer's patients by identifying differential gene expression (mRNA or proteins) in response to a cell-division inhibitor in non-neuronal cells (p. 2, para. 3, p. 12, para. 4 to p. 13, para. 1, claims 3, 6, 10). Markers disclosed comprise CDKN3, cyclin A, cyclin B, p15ink4B, p27kip1, TP53 and others but not the interleukins disclosed in the present patent.

## SUMMARY OF THE INVENTION

The differential sensitivity to cell cycle inhibitors, and in particular the mTOR inhibitor rapamycin, has now been exploited in order to identify new means by which to diagnose and monitor Alzheimer's disease, particularly in the early stages of the disease.

The present invention in its broadest form is defined by the independent claims. The claimed invention involves the gene expression of the genes selected from the group of: interleukin 1 beta (IL-1B); interleukin 2 (IL-2); interleukin 6 (IL-6); and interleukin 10 (IL-10).

References to other markers of tables 1 and 2 in the present specification are if not claimed for reference only.

Therefore, in accordance with a first aspect of the invention, there is provided a method to assist with diagnosis of Alzheimer's disease in a live human subject, which method comprises screening for a differential response to a cell-division inhibitor in non-neuronal cells taken from the human subject as compared with control non-neuronal cells, wherein the differential response is measured by analysing the inhibitor-induced relative changes in gene expression of one or more of the genes selected from the group of: interleukin 1 beta (IL-1B); interleukin 2 (IL-2); interleukin 6 (IL-6); and interleukin 10 (IL-10).

In accordance with a second aspect of the invention, there is provided a method of assessing the risk of Alzheimer's disease progression in a human subject, which method comprises screening for a differential response to a cell-division inhibitor in non-neuronal cells taken from the human subject as compared with control non-neuronal cells, wherein the differential response is measured by analysing the inhibitor-induced relative changes in gene expression of one or more of the genes selected from the group of: interleukin 1 beta (IL-1B); interleukin 2 (IL-2); interleukin 6 (IL-6); and interleukin 10 (IL-10).

The differential response to a cell-division inhibitor in non-neuronal cells taken from a human subject may be determined by:-

(a) inducing cell division in non-neuronal cells taken from the human subject suspected of having Alzheimer's disease;
(b) separating the dividing non-neuronal cells of (a) into two pools and treating one pool of cells with a cell-division inhibitor;
(c) determining the level of expression of one or more of the genes selected from the group of: interleukin 1 beta (IL-1B); interleukin 2 (IL-2); interleukin 6 (IL-6); and interleukin 10 (IL-10) in the pool of cells treated with the cell-division inhibitor and in the untreated pool of cells;
(d) comparing the level of expression of the one or more genes analysed in (c) for the pool of cells treated with the cell-division inhibitor and the untreated pool of cells, in order to determine any inhibitor-induced relative changes in gene expression;
(e) repeating steps (a-)-(d) for control non-neuronal cells;
(f) comparing any inhibitor-induced relative changes in gene expression observed in non-neuronal cells taken from the human subject to be tested with any inhibitor-induced relative changes in gene expression observed in control non-neuronal cells, in order to determine any differences in inhibitor-induced relative gene expression changes between test and control non-neuronal cells, wherein a difference is indicative of Alzheimer's disease.

Preferably, the control non-neuronal cells are derived from a healthy individual exhibiting normal cognitive and/or neuropsychological test results.

The differential response in gene expression observed in non-neuronal cells taken from human subjects with Alzheimer's disease as compared with control non-neuronal cells may be exploited further in order to identify compounds that may be used to treat Alzheimer's disease or to assess pharmacological agents developed for the treatment of Alzheimer's disease in particular individuals.

Thus, in accordance with a third aspect of the invention, there is provided a method of identifying a compound having potential pharmacological activity in the treatment of Alzheimer's disease, which method comprises screening for a differential response to a cell-division inhibitor in non-neuronal cells, which cells exhibit a cell cycle regulatory defect at the G1/S transition, in the presence and absence of a test compound, wherein the differential response is measured by comparing the inhibitor-induced relative changes in gene expression of one or more of the genes selected from the group of: interleukin 1 beta (IL-1B); interleukin 2 (IL-2); interleukin 6 (IL-6); and interleukin 10 (IL-10) with the inhibitor-induced relative changes in gene expression of one or more the genes selected from the group of: interleukin 1 beta (IL-1B); interleukin 2 (IL-2); interleukin 6 (IL-6); and interleukin 10 (IL-10) in control non-neuronal cells, and wherein a compound which results in a reduction in the differential response to the cell-division inhibitor in non-neuronal cells exhibiting a cell cycle regulatory defect at the G1/S transition is identified as having potential pharmacological activity in the treatment of Alzheimer's disease.

In accordance with a fourth aspect of the invention, there is provided a method of determining whether a pharmacological agent is likely to be of benefit in the treatment of Alzheimer's disease in a particular human individual, which method comprises screening for a differential response to a cell-division inhibitor in non-neuronal cells taken from the particular human individual, in the presence and absence of a pharmacological agent, wherein the differential response is measured by comparing the inhibitor-induced relative changes in gene expression of one or more of the genes selected from the group of: interleukin 1 beta (IL-1B); interleukin 2 (IL-2); interleukin 6 (IL-6); and interleukin 10 (IL-10) with the inhibitor-induced relative changes in gene expression of one or more the genes selected from the group of: interleukin 1 beta (IL-1B); interleukin 2 (IL-2); interleukin 6 (IL-6); and interleukin 10 (IL-10) in control non-neuronal cells, and wherein a pharmacological agent which results in a reduction in the differential response to the cell-division inhibitor in non-neuronal cells taken from the human individual is identified as likely to be of benefit in the treatment of Alzheimer's disease in said individual.

In accordance with a fifth aspect of the invention, there is provided a method of monitoring the efficacy of a pharmacological agent in the treatment of Alzheimer's disease in a particular human individual, which method comprises screening for a differential response to a cell-division inhibitor in non-neuronal cells taken from the particular human individual, in the presence and absence of a pharmacological agent, wherein the differential response is measured by comparing the inhibitor-induced relative changes in gene expression of one or more of the genes selected from the group of: interleukin 1 beta (IL-1B); interleukin 2 (IL-2); interleukin 6 (IL-6); and interleukin 10 (IL-10) with the inhibitor-induced relative changes in gene expression of one or more the genes selected from the group of: interleukin 1 beta (IL-1B); interleukin 2 (IL-2); interleukin 6 (IL-6); and interleukin 10 (IL-10) in control non-neuronal cells, and wherein a pharmacological agent which results in a reduction in the differential response to the cell-division inhibitor in non-neuronal cells taken from the human individual is identified as efficacious in the treatment of Alzheimer's disease in said individual.

In all aspects of the invention described above the one or more genes used for measuring relative changes in gene expression are selected from the group of genes consisting of: interleukin 1 beta (IL1B); interleukin 2 (IL-2); interleukin 6 (IL-6); and interleukin 10 (IL-10).

Furthermore, by investigating the differential sensitivity of cells to the inhibitor rapamycin, new biomarkers of the mTOR signalling pathway have been identified that may be used to monitor mTOR signalling in human cells.

Therefore, in a sixth aspect of the present invention, there is provided a method by which to monitor mTOR signalling in a human cell, which method comprises detecting one or more biomarkers of the mTOR signalling pathway, wherein said biomarkers are selected from the rapamycin-sensitive genes selected from the group of: interleukin 1 beta (IL-1B); interleukin 2 (IL-2); interleukin 6 (IL-6); and interleukin 10 (IL-10).

Moreover, in a seventh aspect of the invention, there is provided use of one or more rapamycin-sensitive genes as biomarkers of the mTOR signalling pathway in a human cell wherein said genes are selected from the genes selected from the group of: interleukin 1 beta (IL-1B); interleukin 2 (IL-2); interleukin 6 (IL-6); and interleukin 10 (IL-10).

In all aspects of the invention described above the rapamycin-sensitive genes of interest are selected from the group of genes consisting of: interleukin 1 beta (IL1B); interleukin 2 (IL-2); interleukin 6 (IL-6); and interleukin 10 (IL-10).

## BRIEF DESCRIPTION OF THE FIGURES

Figure 1 Relative rapamycin-induced gene expression changes in the IL 1 beta gene measured in lymphocytes taken from patients with Alzheimer's disease (AD) and control human subjects.
Figure 2 Relative rapamycin-induced gene expression changes in the IL 1 beta gene measured in lymphocytes taken from patients with Alzheimer's disease (AD) and control human subjects, calculated per live cell.

**Figure 3** Relative rapamycin-induced gene expression changes in the IL-2 gene measured in lymphocytes taken from patients with Alzheimer's disease (AD) and control human subjects.

**Figure 4** Relative rapamycin-induced gene expression changes in the IL-2 gene measured in lymphocytes taken from patients with Alzheimer's disease (AD) and control human subjects, calculated per live cell.

**Figure 5** Relative rapamycin-induced gene expression changes in the IL-6 gene measured in lymphocytes taken from patients with Alzheimer's disease (AD) and control human subjects.

**Figure 6** Relative rapamycin-induced gene expression changes in the IL-6 gene measured in lymphocytes taken from patients with Alzheimer's disease (AD) and control human subjects, calculated per live cell.

**Figure 7** Relative rapamycin-induced gene expression changes in the IL-10 gene measured in lymphocytes taken from patients with Alzheimer's disease (AD) and control human subjects.

**Figure 8** Relative rapamycin-induced gene expression changes in the IL-10 gene measured in lymphocytes taken from patients with Alzheimer's disease (AD) and control human subjects, calculated per live cell.

**Figure 9** ROC curve for Alzheimer's disease prediction/diagnosis based on measuring differential expression of IL 1 beta.

**Figure 10** ROC curve for Alzheimer's disease prediction/diagnosis based on measuring differential expression of IL 1 beta and IL-6.

**Figure 11** ROC curve for Alzheimer's disease prediction/diagnosis based on measuring differential expression of IL 1 beta, IL-2, IL-6 and IL-10.

**Figure 12** ROC curve for Alzheimer's disease prediction/diagnosis based on measuring differential expression of IL 1 beta, IL-2, IL-6 and IL-10 and determining ApoE genotype status.

**Figure 13** Correlation between rapamycin-induced changes in IL 1 beta gene expression and rapamycin-induced changes in cell proliferation.

**Figure 14** Correlation between rapamycin-induced changes in IL-10 gene expression and rapamycin-induced changes in cell proliferation.

**Figure 15** Correlation between rapamycin-induced changes in IL-2 gene expression and rapamycin-induced changes in cell proliferation.

**Figure 16** Correlation between rapamycin-induced changes in IL-6 gene expression and rapamycin-induced changes in cell proliferation.

## DETAILED DESCRIPTION

The present invention provides methods for diagnosing Alzheimer's disease in live human subjects. The methods may be used to identify individuals suspected of having this particular neurodegenerative disease or those who are considered at risk of developing clinical symptoms associated with Alzheimer's disease.

As mentioned above, clinical diagnosis of Alzheimer's disease is typically based on evaluation of clinical criteria, such as the NINCDS/ADRDA criteria (McKhann, G. et al., (1984) Neurology 34: 939-944). However, such diagnostic criteria applied in the clinic are based on the measurement of cognitive parameters, and are thus reliant on the onset of cognitive symptoms in patients with this disease.

It is now clear from extensive research that the pathophysiological changes defining Alzheimer's disease are detectable in individuals years before these patients show any signs of cognitive impairment. Alzheimer's disease has accordingly, been classified into three phases:

(i) asymptomatic Alzheimer's disease (preclinical stage);
(ii) mild cognitive impairment (MCI) due to Alzheimer's disease (pre-dementia stage); and
(iii) clinically-defined Alzheimer's disease (dementia).

In the context of the present invention, the term "diagnosis of Alzheimer's disease" is used very broadly and should be taken to mean diagnosis of an individual having disease at any one of the three phases defined above. In one embodiment, the method of the invention is used to diagnose or assist with diagnosis of Alzheimer's disease in its preclinical stage in an individual with no symptoms of disease, for example no signs of cognitive impairment. In other embodiments, the same basic methodology may be used to screen subjects who are "symptomatic" to varying degrees. For example, the method of the invention may be applied to individuals classified according to standard criteria, for example the Mayo Clinic diagnostic criteria (Winblad et al., (2004) J. Intern. Med 256: 240-246), as having mild cognitive impairment (MCI). Not all patients classified as having MCI will have the type of underlying neurodegeneration associated with Alzheimer's disease. Thus, the present method may be used to distinguish or assist with distinguishing between individuals with MCI that have underlying Alzheimer's disease and therefore are likely to go on to develop Alzheimer's disease-associated dementia, and those that have MCI attributable to a different cause or condition. In a further embodiment of the invention, the method may be used to diagnose or assist with diagnosis of Alzheimer's disease in a human subject exhibiting one or more symptoms consistent with Alzheimer's disease.

The diagnostic methods of the present invention may also be used in conjunction with existing diagnostic criteria, for example the NINCDS/ADRDA criteria, in order to verify or substantiate an Alzheimer's disease diagnosis in a human subject who already meets the existing criteria for a positive diagnosis. In this embodiment, the present methods may provide an adjunct to alternative diagnostic tests, wherein the present methods are independent of neuropsychological symptoms. This may allow for a more reliable diagnosis of clinical Alzheimer's disease, particularly since not all patients presenting with dementia symptoms will have Alzheimer's disease as the underlying cause.

The present methods are used in particular, to assist with diagnosis of Alzheimer's disease in a live human subject. A *definitive* diagnosis of Alzheimer's disease is generally considered by those in the field to be impossible in a live subject, and can only be made post-mortem following pathological examination of brain tissue from the patient. Thus, although present methods may seek to "diagnose" Alzheimer's disease in live subjects, such a diagnosis is typically based on an assessment of the likelihood that any given individual has the disease. In this regard, individuals may be classified as "possible Alzheimer's disease" or "probable Alzheimer's disease" based on the results of current diagnostic tests.

The present methods may therefore be used to "assist with diagnosis" meaning that they are used to assess the likelihood that an individual has Alzheimer's disease at any one of the three phases of the disease described above. In preferred embodiments, the present method may be used to assist with diagnosis of early-stage Alzheimer's disease in asymptomatic patients or patients exhibiting mild cognitive impairment.

The methods of the invention may also be used in combination or together with other methods or tests used for Alzheimer's disease diagnosis, for example in order to improve the specificity and/or sensitivity of these methods or tests. In specific embodiments, the present methods may be carried out in combination with a test designed to monitor one or more biomarkers of Alzheimer's disease in a particular individual, and the combined result may be used to assess the likelihood that the individual has Alzheimer's disease. In alternative embodiments, the present method may be used to independently substantiate the results of other diagnostic tests.

In preferred embodiments, the methods of the invention may be carried out in combination with methods used to determine the ApoE status of individuals. More specifically, the gene encoding ApoE is polymorphic, with three major alleles *ApoE2*, *ApoE3* and *ApoE4*, which translate into three major isoforms of the protein (apoE2, apoE3 and apoE4). The *ApoE4* allele is a known genetic risk factor for Alzheimer's disease in a variety of ethnic groups and can account for approximately 50% of cases in many populations (Waring SC and Rosenberg RN. Genome-Wide Association Studies in Alzheimer Disease. Arch Neurol 2008;65(3):329-334). Individuals with either one or two copies of *ApoE4* have a higher risk of developing Alzheimer's disease, compared with carriers of the other isoforms. *ApoE4* also reduces the median age of Alzheimer's disease onset from 84 in non-carriers to 68 in homozygotes (Cedazo-Minguez A. Apolipoprotein E and Alzheimer's disease: molecular mechanisms and therapeutic opportunities. J. Cell. Mol. Med. 2007;11(6):1227-1238).

Although the *ApoE4* allele is an established genetic risk factor for Alzheimer's disease, this marker is not reliable on its own for the diagnosis of Alzheimer's disease in a clinical setting. Therefore, in preferred embodiments, the results obtained using the presently-claimed methods may be combined with the results obtained from ApoE genotyping of the same individual to diagnose or assist with diagnosis of Alzheimer's disease. The human subject may be screened for the presence of at least one *ApoE4* allele.

The present method is intended to provide a means to diagnose and/or assist with diagnosis of Alzheimer's disease in multiple settings. In one embodiment, the present method may be used to diagnose individuals with Alzheimer's disease so as to identify patients suitable for the assessment of new Alzheimer's disease treatments, for example the identification of suitable subjects for clinical trials. New treatments or therapies designed to be preventive and/or curative may only have the best chance of success in patients with asymptomatic or early-stage disease. The present method may therefore be used to diagnose or assist with diagnosis of pre-clinical Alzheimer's disease in asymptomatic individuals or to diagnose or assist with diagnosis of individuals with MCI that have underlying Alzheimer's disease pathology, for the purposes of assessing new treatments specifically in these patients. As improved treatments for Alzheimer's disease become available, the present methods may also be used to diagnose or assist with diagnosis of individuals so as to identify patients who will benefit from treatments that may have the ability to prevent cognitive decline.

The present invention also provides methods of assessing the risk of Alzheimer's disease progression in a human subject. In this context, "Alzheimer's disease progression" should be taken to mean the progressive neurodegeneration associated with this disease and/or the progressive decline in cognitive function that accompanies the underlying neuropathology. Such methods may be applied to individuals suspected of having any one of the three phases of Alzheimer's disease defined above, or individuals considered at risk of developing this disease.

In preferred embodiments of the invention, the methods provide means by which to assess or predict cognitive decline in human subjects by identifying individuals with early-stage Alzheimer's disease who will go on to develop Alzheimer's disease-associated dementia. These prognostic methods, particularly for patients suspected to be "at risk" or in the early stages of Alzheimer's disease, are made possible by the fact that the present methodology detects pathophysiological changes preceding the development of recognisable clinical symptoms. In certain embodiments, the individual or human subject for testing will be asymptomatic for Alzheimer's disease. In alternative embodiments, the individual or human subject for testing will exhibit mild cognitive impairment or will exhibit one or more symptoms consistent with Alzheimer's disease.

The methods of the invention for assessing the risk of Alzheimer's disease progression may also be used in combination or together with other methods or tests used to assess Alzheimer's disease progression, for example in order to improve the specificity and/or sensitivity of these methods or tests. In certain embodiments, the results obtained using the presently-claimed methods may be combined with the results obtained from ApoE genotyping of the same individual to assess the risk of Alzheimer's disease progression.

Embodiments of the methods of the present invention described above comprise screening for a differential response to a cell division inhibitor in non-neuronal cells taken from a human subject. In the following passages, features of this screening process will be described in further detail. It is to be understood that features described as being preferred or advantageous are equally applicable to all aspects and/or methods of the present invention involving screening for a differential response to a cell division inhibitor in non-neuronal cells. Furthermore, any feature described as preferred or advantageous may be combined with any other feature so-described, unless it is stated otherwise.

Embodiments of the methods of the invention are most preferably carried out *in vitro* on non-neuronal cells isolated from the human subject. Any non-neuronal cell type which exhibits a differential response to a cell division inhibitor in patients with Alzheimer's disease may be isolated and used for analysis. It is preferred to use cells that are readily obtainable from the subject for obvious practical reasons. In one embodiment, the method is carried out on lymphocytes isolated from the subject and cultured *in vitro*. The use of lymphocytes is particularly convenient since they are easily obtained from a blood sample. Another embodiment involves the use of fibroblasts, particularly skin fibroblasts, which may be conveniently obtained from a skin biopsy.

The non-neuronal cells may be screened for their response to any cell division inhibitor. In preferred embodiments of the invention, the cell-division inhibitor may be any G1 inhibitor *i.e.* an inhibitor that brings about cell cycle arrest during the G1 phase of the cell cycle. In further preferred embodiments, the cell division inhibitor is an inhibitor of the serine/threonine kinase mTOR, with the mTOR inhibitor rapamycin being particularly preferred.

In certain embodiments, a "differential response" to a cell-division inhibitor in non-neuronal cells taken from a human subject to be tested, is measured by essentially a three-stage process. First, any inhibitor-induced relative gene expression changes occurring in non-neuronal cells taken from the test subject are determined by analysing separately the levels of gene expression in non-neuronal cells from the test subject treated with inhibitor (the treated sample) and non-neuronal cells from the test subject that are not treated with inhibitor (the untreated sample), and comparing the levels of gene expression for treated and untreated samples in order to calculate any inhibitor-induced relative gene expression changes.

In the second stage, the process described in the first step for determining any inhibitor-induced relative gene expression changes in the non-neuronal cells taken from the human subject to be tested, is repeated using control non-neuronal cells. As used herein, "control non-neuronal cells" are non-neuronal cells typically derived from a healthy individual exhibiting normal cognitive and/or neuropsychological test results. It is preferred that the control non-neuronal cells are the same cell type as the non-neuronal cells taken from the human subject to be tested. It is also preferred that the control cells are derived from an age-matched healthy individual. The result of the second step is the determination of any inhibitor-induced relative gene expression changes occurring in control cells.

In the third, final stage, any inhibitor-induced relative gene expression changes observed in the non-neuronal cells taken from the human subject to be tested are compared with any inhibitor-induced relative gene expression changes observed in the control non-neuronal cells in order to determine any differences. If any differences are observed between the inhibitor-induced relative gene expression changes in the non-neuronal cells taken from the human subject as compared with the control non-neuronal cells, this is classified as a "differential response" to the cell-division inhibitor, and a differential response is indicative of Alzheimer's disease.

In particularly preferred embodiments, a "differential response" to a cell-division inhibitor in non-neuronal cells taken from a human subject to be tested is determined in the following way.

In a first step, cell division is induced in non-neuronal cells taken from a human subject suspected of having Alzheimer's disease. The non-neuronal cells may be induced to divide by the addition of any mitogenic stimulus, for example one or more growth factors. If the method is carried out using lymphocytes, then phytohaemagglutinin (PHA) may be used to induce cell division. The cells will typically be cultured in the presence of a mitogenic stimulus for a period of between 12 and 96 hours, preferably a period between 24 and 72 hours, and most preferably a period of 48 hours, prior to any further treatment.

In a second step, the dividing non-neuronal cells exposed to the mitogenic stimulus are separated into two "pools" and one of these pools of cells is treated with a cell-division inhibitor (referred to herein as the "treated cells"). In this context, a "pool" of cells should be taken to mean a discrete culture of cells. Typically, the pool of cells not treated with inhibitor (referred to herein as the "untreated cells") will undergo continued growth in standard culture media.

The rationale behind having "treated" and "untreated" cell pools or cultures is so as to be able to determine the levels of gene expression of one or more genes in the treated and untreated samples and thereafter compare these results. Thus, the relative sizes of the "treated" and "untreated" cell cultures, and/or the quantity of cells within each pool or culture will depend on the quantity of cellular material needed for the subsequent analysis of gene expression. Typically, the non-neuronal cells taken from the test human subject and initially induced to divide by the addition of a mitogenic stimulus, will be divided into two pools or cell cultures of equal size, and an inhibitor, preferably rapamycin, will be added to one of the pools.

Following the addition of inhibitor, the treated and untreated cells may be cultured for any suitable length of time prior to the third step of gene expression analysis. In a preferred embodiment of the invention, both inhibitor-treated and untreated cells are cultured for a period of 24 hours following addition of the inhibitor and prior to harvesting for gene expression analysis.

For both the inhibitor-treated and untreated cells, the level of gene expression of one or more genes is determined. The genes to be analysed are typically endogenous human genes. In the context of the present invention, the term "gene expression" is intended to mean the process by which a DNA or cDNA template is transcribed to produce mRNA and/or the process by which the mRNA is translated in order to produce the corresponding protein. Thus, gene expression may be analysed at the mRNA or protein level using standard techniques known to those skilled in the art.

Suitable methods for the detection/quantitation of mRNAs which may be used in accordance with the present methods are well known in the art, and include, but are not limited to, hybridisation techniques, such as Northern blotting or microarray technologies, and amplification-based techniques such as RT-PCR or nucleic-acid sequence-based amplification (NASBA).

In preferred embodiments of the invention, gene expression is analysed by assessing levels of the corresponding protein. Suitable techniques for assessing protein levels are known in the art and include, but are not limited to flow cytometry, immunoblot analysis, ELISA, Elispot and Fluorospot assays. In certain embodiments, these assays may be used in conjunction with commercially-available antibodies that bind to the protein of interest, in order to determine protein levels.

In a fourth step, the "relative changes", in inhibitor-induced gene expression may be determined by calculating the difference in either mRNA transcript levels or the levels of the corresponding protein, for the genes of interest, in inhibitor-treated versus inhibitor-untreated non-neuronal cells.

In order to determine whether or not there is a "differential response" to an inhibitor in non-neuronal cells taken from a human subject suspected of having Alzheimer's disease, the relative changes in inhibitor-induced gene expression calculated for the non-neuronal cells taken from the test subject are compared with any relative changes in inhibitor-induced gene expression calculated using control non-neuronal cells.

As mentioned above, "control non-neuronal cells" are non-neuronal cells typically derived from a healthy individual exhibiting normal cognitive and/or neuropsychological test results. Thus, the process for determining relative changes in inhibitor-induced gene expression detailed above for non-neuronal cells taken from a test subject is repeated using control non-neuronal cells.

Once inhibitor-induced relative gene expression changes have been determined for both non-neuronal cells from the human subject to be tested ("test") and control non-neuronal cells ("control"), the final step is to compare the gene expression changes for the "test" and "control" in order to detect any differences. This calculation or identification of differences in any inhibitor-induced relative gene expression changes via a comparison of the result(s) obtained using non-neuronal cells from a test human subject (test non-neuronal cells) and the result(s) obtained using control non-neuronal cells provides the final result needed to assist with diagnosis of Alzheimer's disease and/or assess the risk of Alzheimer's disease progression or cognitive decline. In particular, a difference between test non-neuronal cells and control non-neuronal cells is indicative of Alzheimer's disease.

The degree of "difference" considered significant for the purposes of a positive test outcome may be established according to the sensitivity and /or specificity requirements of those utilising the methods described herein for diagnostic and/or monitoring purposes. Moreover, different "cutoffs" or levels of difference may be used to stratify subjects based on their relative risk, for example, the relative likelihood that a given individual will have Alzheimer's disease.

The present invention in its broadest form is defined by the independent claims. The claimed invention involves the gene expression of the genes selected from the group of: interleukin 1 beta (IL-1B); interleukin 2 (IL-2); interleukin 6 (IL-6); and interleukin 10 (IL-10).

References to other markers of tables 1 and 2 in the present specification are if not claimed for reference only.

In the methods of the present invention, gene expression changes are measured by monitoring the expression of one or more of the genes shown in Tables 1 and 2. The 31 genes shown in Table 1 were found to be down-regulated in lymphocytes in response to rapamycin treatment, and the 73 genes shown in Table 2 were found to be up-regulated in lymphocytes in response to rapamycin treatment.

The genes shown in both Tables encode proteins that are either secreted by cells or are typically located in the cytoplasm. There are clearly practical advantages associated with methods involving measurement of the relative expression of proteins, wherein such proteins are readily detected by virtue of their extracellular and/or cytoplasmic location. For example, secreted proteins can be readily detected by ELISA or similar assays such as the Elispot assay or the related Fluorospot assay. Thus, by exploiting differential sensitivity to cell cycle inhibitors, and in particular, the inhibitor rapamycin, and thereby identifying genes regulated downstream of such inhibitors, the present methods significantly simplify the process by which the differential response to a cell-division inhibitor may be assessed in non-neuronal cells.

The rapamycin-sensitive genes shown in Tables 1 and 2 encode a variety of different types of protein including cytokines, enzymes, growth factors, kinases, peptidases, phosphatases and transporter proteins. The proteins of Table 1 have been implicated in a diverse array of molecular and cellular functions including cell-to-cell signalling, antigen presentation, cellular movement, cell death and cellular growth and proliferation. The proteins of Table 2 are similarly diverse and have been implicated in cellular growth and proliferation, cell morphology, cellular development, antigen presentation and molecular transport.

Furthermore, the proteins encoded by the genes shown in Table 1 have been associated with a variety of diseases and disorders including haematological disease, infectious disease, inflammatory responses, endocrine system disorders and metabolic disease. Similarly, the proteins encoded by the genes shown in Table 2 have been associated with endocrine system disorders, genetic disorders, metabolic disease, cardiovascular disease and cancer.

The proteins encoded by the genes shown in both Tables have been implicated in the development and function of various physiological systems. These include haematological system development and function/hematopoiesis, immune cell trafficking, tissue development and morphology and lymphoid tissue structure and development.

The methods of the present invention may involve measuring the inhibitor-induced relative changes in expression for one or more, two or more, three or more, four or more and up to 104 of the genes shown in Tables 1 and 2. Wherein a combination of genes is to be tested, these genes may be selected only from Table 1 or only from Table 2 or may be selected from the genes shown in both Tables.

In preferred embodiments, the methods of the present invention involve measuring the inhibitor-induced relative changes in expression of one or more genes selected from: interleukin 1 beta (IL1B); interleukin 2 (IL-2); interleukin 6 (IL-6); and interleukin 10 (IL-10). In further preferred embodiments, the methods of the present invention involve measuring the inhibitor-induced relative changes in expression of two or more genes wherein a first gene is interleukin 1 beta (IL1B) and at least one other gene is selected from: interleukin 2 (IL-2); interleukin 6 (IL-6); and interleukin 10 (IL-10). In further preferred embodiments, the methods of the present invention involve measuring the inhibitor-induced relative changes in expression of interleukin 1 beta (IL1B), interleukin 2 (IL-2), interleukin 6 (IL-6) and interleukin 10 (IL-10). In these preferred embodiments of the invention, gene expression is preferably measured by analysing levels of the corresponding protein produced from the IL1B, IL-2, IL-6 and/or IL-10 genes, for example by ELISA. In such embodiments, the cell-division inhibitor is preferably rapamycin.

Wherein the methods of the invention involve measuring changes in gene expression for more than one gene shown in Table 1 or 2, the expression of each gene may be measured via a separate assay. Alternatively, changes in expression of multiple genes may be measured simultaneously using a nucleic acid microarray, for example to detect multiple mRNA species, or a protein or antibody array, to detect multiple proteins. In the context of the present invention, the term 'multiple' should be taken to mean at least two, at least three, at least four and so forth.

The methods of the present invention described above may be used for diagnosing and/or assessing any individuals suspected of having Alzheimer's disease or considered to be at risk of developing this disease. In preferred embodiments, the methods are used to diagnose and/or assess individuals suspected of having sporadic Alzheimer's disease. In other embodiments, the methods of the present invention may be applied to subjects suspected of having familial forms of Alzheimer's disease.

In a further aspect, the present invention also provides methods for identifying a compound having potential pharmacological activity in the treatment of Alzheimer's disease. Such methods also involve screening for a differential response to a cell-division inhibitor in non-neuronal cells ("test" cells), and in particular non-neuronal cells from Alzheimer's disease patients which exhibit a cell cycle regulatory defect at the G1/S transition. The differential response is measured by comparing the relative changes in gene expression of one or more of the genes shown in Tables 1 and 2 as determined for the "test" cells with the relative changes in gene expression of one or more of the genes shown in Tables 1 and 2 as determined for control non-neuronal cells. All embodiments of the screening process described in respect of the diagnostic and prognostic methods detailed above apply *mutatis mutandis* to this further aspect of the invention and therefore are not repeated for reasons of conciseness.

In order to use the screening process described above to identify a compound having potential pharmacological activity in the treatment of Alzheimer's disease, it may be necessary to monitor the differential response of the test non-neuronal cells, which exhibit a cell cycle regulatory defect at the G1/S transition, both before and after exposure to the test compound. In preferred embodiments, the test non-neuronal cells will have been previously isolated from a human subject known to have or suspected of having Alzheimer's disease and the cells will thereafter be exposed to the test compound for the purposes of *in vitro* testing.

In order to determine whether a test compound has an effect on the differential response to a cell-division inhibitor observed in non-neuronal cells exhibiting a regulatory defect in the G1/S cell cycle transition, the following stages of analysis may be carried out. In a first stage, "test non-neuronal cells" that have not been exposed to the test compound, are compared with "control non-neuronal cells" that have also not been exposed to the test compound and have been isolated from a healthy individual. This comparison involves determining any inhibitor-induced relative gene expression changes for both the "test" and "control" non-neuronal cells using the methodology detailed.above, and thereafter comparing the results to determine any "differential response" to the inhibitor. In a second stage, "test non-neuronal cells", that have been exposed to the test compound, are compared with "control non-neuronal cells" that have not been exposed to the test compound, and any differences in inhibitor-induced relative gene expression changes observed are used to determine any "differential response". In a third stage, any differential response measured in the first stage of the analysis is compared with any differential response measured in the second stage of the analysis. If, in the second stage, following exposure of the test non-neuronal cells to the test compound, any differential response detected (by comparing "test" and "control" non-neuronal cells) is reduced, then the test compound is identified as having potential pharmacological activity in the treatment of Alzheimer's disease.

The methods described above may be used to screen any compounds known to those skilled in the art including small molecule inhibitors and biological agents such as antibodies. In preferred embodiments, the test compounds are not known for the treatment of Alzheimer's disease. Moreover, embodiments of the methods may involve testing panels or libraries of test compounds for their potential as pharmacological agents in the treatment of Alzheimer's disease.

In a further aspect, the present invention provides a method of determining whether a pharmacological agent is likely to be of benefit in the treatment of Alzheimer's disease in a particular human individual. Embodiments of the earlier aspects of the present invention also apply *mutatis mutandis* to this further aspect.

In this aspect, the suitability of a particular pharmacological agent for a particular individual is assessed by screening for a differential response to a cell-division inhibitor in non-neuronal cells taken from the particular human individual, in the presence and absence of a pharmacological agent, wherein the differential response is measured by comparing the inhibitor-induced relative changes in gene expression of one or more of the genes shown in Tables 1 and 2 with the inhibitor-induced relative changes in gene expression of one or more the genes shown in Tables 1 and 2 in control non-neuronal cells.

In preferred embodiments, the screening methods of the present invention involve measuring the inhibitor-induced relative changes in expression of one or more genes selected from: interleukin 1 beta (IL1B); interleukin 2 (IL-2); interleukin 6 (IL-6); and interleukin 10 (IL-10). In further preferred embodiments, the screening methods of the present invention involve measuring the inhibitor-induced relative changes in expression of two or more genes wherein a first gene is interleukin 1 beta (IL1B) and at least one other gene is selected from: interleukin 2 (IL-2); interleukin 6 (IL-6); and interleukin 10 (IL-10). In further preferred embodiments, the methods of the present invention involve measuring the inhibitor-induced relative changes in expression of interleukin 1 beta (IL1B), interleukin 2 (IL-2), interleukin 6 (IL-6) and interleukin 10 (IL-10). In these preferred embodiments of the invention, gene expression is preferably measured by analysing levels of the corresponding protein produced from the IL1B, IL-2, IL-6 and/or IL-10 genes, for example by ELISA. In such embodiments, the cell-division inhibitor is preferably rapamycin.

In certain embodiments, the pharmacological agent for testing may be a known treatment for Alzheimer's disease, in particular a known G1/S cell cycle inhibitor. Moreover, the pharmacological agent for testing may be administered directly to the human subject such that the cells are exposed to the pharmacological agent *in vivo.* Under these circumstances, non-neuronal cells would be harvested from the individual before and after treatment. Alternatively, the non-neuronal cells may be first isolated from the human subject and thereafter tested both before and after exposure to the pharmacological agent *in vitro.* A pharmacological agent which reduces the differential response observed in the non-neuronal cells taken from the individual following treatment is identified as likely to be of benefit in the treatment of that particular individual.

In a yet further aspect of the invention, there is provided a method of monitoring the efficacy of a pharmacological agent in the treatment of Alzheimer's disease in a particular human individual. Such methods may be used to assess new pharmacological agents that are being tested in the context of clinical trials. Alternatively, such methods may be used to assess the clinical progress of a particular individual receiving a pharmacological agent predicted to be efficacious on the basis of *in vitro* results or a pharmacological agent known to have been used for the successful treatment of other individuals with Alzheimer's disease.

In order to assess the efficacy of a pharmacological agent in the treatment of Alzheimer's disease in a particular individual or patient, the following stages of analysis may be carried out. In a first step, a "test" non-neuronal cell sample is taken from the individual before treatment commences. This first test sample is analysed for any differential response to a cell-cycle inhibitor using the screening process described above. In particular, this involves comparing any inhibitor-induced relative gene expression changes in one or more of the genes shown in Tables 1 or 2 with any inhibitor-induced relative gene expression changes in one or more of the genes shown in Tables 1 or 2 as determined for control non-neuronal cells, wherein the control non-neuronal cells derive from a healthy individual. Once the individual's treatment has commenced, further "test" samples may be taken at various time intervals, and these test samples will be analysed for any differential response to a cell-cycle inhibitor on the basis of comparison with control non-neuronal cells. Thereafter, the differential responses observed over time may themselves be compared in order to determine whether the pharmacological agent is causing a reduction over time in any differential response to an inhibitor, wherein a reduction is indicative of a positive response to the pharmacological agent in the particular individual being treated. The results of this type of assessment may be used to assess an individual's clinical progress in response to a particular pharmacological agent, and thereby inform decisions regarding the patient's ongoing treatment regimen.

As discussed elsewhere herein, the genes to be analysed in the context of the present methods may be selected from the rapamycin-sensitive genes shown in Tables 1 and 2. The inhibitor rapamycin is known to inhibit the serine/threonine kinase mTOR in human cells, and thereby reduce signalling downstream of this protein.

Therefore, in a further aspect, the present invention also provides a method by which to monitor mTOR signalling in a human cell, which method comprises detecting one or more biomarkers of the mTOR signalling pathway, wherein said biomarkers are selected from the rapamycin-sensitive genes shown in Tables 1 and 2.

In preferred embodiments, the biomarkers are selected from the group of rapamycin-sensitive genes consisting of: interleukin 1 beta (IL1B); interleukin 2 (IL-2); interleukin 6 (IL-6); and interleukin 10 (IL-10).

The kinase, mTOR, functions within the context of two cytoplasmic protein complexes known as mTORC1 and mTORC2. It is however, only the mTORC1 complex that is sensitive to the inhibitor rapamycin. Thus, in a preferred embodiment of this aspect of the invention, there is provided a method by which to monitor mTORC1 signalling in a human cell.

The cytoplasmic kinase mTOR is stimulated or activated by a wide variety of upstream signals. These include signals generated or triggered as a result of nutrient sensing, hypoxia, and/or the activity of growth factors and their cognate receptors. Activation of mTOR upregulates its kinase activity and thereby increases mTOR-mediated phosphorylation of downstream protein targets within the cell. In most cases, the direct downstream protein targets of mTOR interact with a variety of further molecular targets, and in doing so, stimulate a wide variety of cellular responses, such as increased protein synthesis and the promotion of cell growth and proliferation. The chain of molecular events triggered downstream of mTOR-mediated phosphorylation of its direct protein targets is defined herein as the "mTOR signalling pathway".

The methods of the present aspect of the invention allow for the monitoring of mTOR signalling. By "monitoring" is meant determination of the level of activity downstream of the mTOR kinase, for example the level of activity of proteins present within the downstream signalling pathways. As used herein, "monitoring" should also be taken to mean the determination of changes in the level of activity of proteins within the mTOR pathway, for example in the presence and absence of a chemical compound or pharmacological agent, and determination of the functional integrity of the mTOR signalling pathway within a human cell.

In the present aspect, "biomarkers" of the mTOR signalling pathway are used to characterise or monitor mTOR signalling in human cells. The term "biomarker" is used herein broadly to mean any molecule whose level correlates with the activity of a molecular or cellular pathway, in this case the mTOR signalling pathway. The use of such a biomarker provides for a surrogate measure of activity through the pathway of interest. The present methods may comprise detection of one or more, two or more, three of more, four or more and up to 104 biomarkers of the mTOR signalling pathway.

In the context of the present invention, the biomarkers of the mTOR signalling pathway are selected from the rapamycin-sensitive genes shown in Tables 1 and 2, and in particular, the expression products thereof. In a preferred embodiment, the biomarkers of the mTOR signalling pathway for use in the methods of the present invention are selected from the proteins encoded by the rapamycin-sensitive genes shown in Tables 1 and 2. Wherein a combination of biomarkers is to be tested, these biomarkers may be selected from only Table 1, only Table 2, or may be selected from both Tables.

In preferred embodiments, the biomarkers of the mTOR signalling pathway for use in the methods of the present invention are selected from the group of rapamycin-sensitive genes consisting of: interleukin 1 beta (IL1B); interleukin 2 (IL-2); interleukin 6 (IL-6); and interleukin 10 (IL-10), and optionally the proteins encoded thereby.

The 31 genes shown in Table 1 were found to exhibit down-regulated expression in lymphocytes in response to rapamycin treatment, and the 73 genes shown in Table 2 were found to exhibit up-regulated expression in lymphocytes in response to rapamycin treatment. Thus, in a preferred embodiment of the invention, the present method is used to detect decreases in mTOR signalling in a human cell and the method involves determining a decrease in one or more of the biomarkers represented in Table 1 and/or an increase in one or more of the biomarkers represented in Table 2. In a further preferred embodiment of the invention, the present method is used to detect increases in mTOR signalling in a human cell and the method involves determining an increase in one or more of the biomarkers represented in Table 1 and/or a decrease in one or more of the biomarkers represented in Table 2.

The skilled person will appreciate that the technique used to "detect biomarkers" will depend on the exact nature of the biomarker molecule. For example, if the biomarker is a protein encoded by any one of the genes shown in Tables 1 or 2, detection may be carried out using any of the techniques known in the art for detecting proteins, for example immunoblotting, flow cytometry, ELISA, Elispot, Fluorospot and so forth.

Wherein the biomarker to be detected is the mRNA species encoded by any of the genes shown in Tables 1 and 2, suitable techniques for mRNA detection may be used. Various techniques are well known in the art and include hybridisation techniques, such as Northern blotting or microarray technologies, and amplification-based techniques such as RT-PCR or nucleic-acid sequence-based amplification (NASBA).

The methods of the present invention may be used to monitor mTOR signalling in any type of human cell. For example, the human cell may be a cell pre-treated with a compound such as a pharmacological inhibitor. In a preferred embodiment of the invention, the human cell is a lymphocyte.

Furthermore, in preferred embodiments of the invention, the human cell may be taken from an individual or human subject suspected of having a particular condition or disease, or considered to be at risk of developing a particular disease, most preferably Alzheimer's disease. Wherein the human subject is suspected of having or developing Alzheimer's disease, the cell may be taken from a subject that is asymptomatic for Alzheimer's disease, or a subject who exhibits mild cognitive impairment or a subject exhibiting one or more symptoms consistent with Alzheimer's disease.

The purpose of monitoring mTOR signalling in a cell taken from an individual or subject suspected of having a particular disease or considered at risk of a particular disease, may be to assist with diagnosis or prognosis of disease in the subject. In the preferred embodiment of the present invention wherein the human cell is isolated from a human subject suspected of having Alzheimer's disease, the method may be carried out in order to assist with the diagnosis of Alzheimer's disease. It is particularly preferred that the present method is used to assist with the early-stage diagnosis of Alzheimer's disease in either asymptomatic subjects or subjects exhibiting mild cognitive decline.

As detailed above, neuronal cells and some non-neuronal cells from subjects with Alzheimer's disease exhibit defects in cell cycle control, and thus have been found to respond differentially to cell division inhibitors, for example rapamycin. The present method may therefore be used to monitor mTOR signalling in non-neuronal cells, preferably lymphocytes, taken from a human subject suspected of having Alzheimer's disease following treatment with a cell division inhibitor, preferably rapamycin, in order to detect any differential response in the non-neuronal cells of the human subject as compared with control non-neuronal cells.

The present methods may also be used to diagnose and/or assist with diagnosis of other diseases or conditions wherein dysregulation of signalling through the mTOR pathway is an underlying cause or consequence, for example cancer, type II diabetes, dementia following brain injury or stroke.

In a further aspect of the invention, there is provided use of one or more rapamycin-sensitive genes as biomarkers of the mTOR signalling pathway in a human cell wherein said genes are selected from the genes shown in Tables 1 and 2. All embodiments described in respect of the methods for monitoring mTOR signalling in a human cell apply *mutatis mutandis* to the use aspect of the invention recited above and therefore are not repeated for reasons of conciseness.

**Table 1 Down-regulated genes**

| ID | Symbol | Entrez Gene Name | Location | Type(s) | q value | Fold change |
|---|---|---|---|---|---|---|
| ACSL6 | ACSL6 | acyl-CoA synthetase long-chain family member 6 | Cytoplasm | enzyme | 0.350051 | -1.79485 |
| CROT | CROT | carnitine O-octanoyltransferase | Cytoplasm | enzyme | 0.386898 | -2.4978 |
| EGLN3 | EGLN3 | egl nine homolog 3 (C. elegans) | Cytoplasm | enzyme | 4.876128 | -1.95857 |
| FUT9 | FUT9 | fucosyltransferase 9 (alpha (1,3) fucosyltransferase) | Cytoplasm | enzyme | 2.127555 | -1.64844 |
| GPAM | GPAM | glycerol-3-phosphate acyltransferase, mitochondrial | Cytoplasm | enzyme | 9.500153 | -1.23142 |
| MOXD1 | MOXD1 | monooxygenase, DBH-like 1 | Cytoplasm | enzyme | 0.755247 | -2.42089 |
| PTGS2 | PTGS2 | prostaglandin-endoperoxide synthase 2 (prostaglandin G/H synthase and cyclooxygenase) | Cytoplasm | enzyme | 8.108912 | -1.2871 |
| PYCR1 | PYCR1 | pyrroline-5-carboxylate reductase 1 | Cytoplasm | enzyme | 4.864678 | -1.29403 |

(continued)

| ID | Symbol | Entrez Gene Name | Location | Type(s) | q value | Fold change |
|---|---|---|---|---|---|---|
| RRAD | RRAD | Ras-related associated with diabetes | Cytoplasm | enzyme | 9.443493 | -1.63179 |
| ST6GAL NAC2 | ST6GAL NAC2 | ST6 (alpha-N-acetyl-neuram inyl-2,3-beta-galactosyl-1,3)-N-acetylgalactosaminide alpha-2,6-sialyltransferase 2 | Cytoplasm | enzyme | 7.768988 | -1.24228 |
| UBE2M | UBE2M | ubiquitin-conjugating enzyme E2M (UBC12 homolog, yeast) | Cytoplasm | enzyme | 8.787849 | -1.50361 |
| HCK | HCK | hemopoietic cell kinase | Cytoplasm | kinase | 9.500153 | -1.40301 |
| PRKG1 | PRKG1 | protein kinase, cGMP-dependent, type I | Cytoplasm | kinase | 9.544845 | -1.11593 |
| DYNC2L1 1 | DYNC2LI 1 | dynein, cytoplasmic 2, light intermediate chain 1 | Cytoplasm | other | 6.334793 | -1.90235 |
| SGTA | SGTA | small glutamine-rich tetratricopeptide repeat (TPR)-containing, alpha | Cytoplasm | other | 9.500153 | -1.40315 |
| INPP5E | INPP5E | inositol polyphosphate-5-phosphatase, 72 kDa | Cytoplasm | phosphat ase | 6.62909 | -1.4981 |
| AP1S1 | AP1S1 | adaptor-related protein complex 1, sigma 1 subunit | Cytoplasm | transporter | 8.493268 | -1.48813 |
| CCL2 | CCL2 | chemokine (C-C motif) ligand 2 | Extracellular Space | cytokine | 0 | -2.66056 |
| CCL8 | CCL8 | chemokine (C-C motif) ligand 8 | Extracellular Space | cytokine | 0.301273 | -2.62023 |
| IL10 | IL10 | interleukin 10 | Extracellular Space | cytokine | 0.301273 | -2.72661 |
| IL2 | IL2 | interleukin 2 | Extracellular Space | cytokine | 8.787849 | -1.40663 |
| IL3 | IL3 | interleukin 3 (colony-stimulating factor, multiple) | Extracellular Space | cytokine | 0.338759 | -2.12321 |
| IL6 | IL6 | interleukin 6 (interferon, beta 2) | Extracellular Space | cytokine | 9.500153 | -1.3333 |
| LOXL3 | LOXL3 | lysyl oxidase-like 3 | Extracellular Space | enzyme | 8.542884 | -1.3654 |
| FGF18 | FGF18 | fibroblast growth factor 18 | Extracellular Space | growth factor | 8.787849 | -1.25471 |
| FGF2 | FGF2 | fibroblast growth factor 2 (basic) | Extracellular Space | growth factor | 9.762848 | -2.24068 |
| ADAMTS 16 | ADAMTS 16 | ADAM metallopeptidase with thrombospondin type 1 motif, 16 | Extracellular Space | other | 9.443493 | -1.49705 |
| COL8A2 | COL8A2 | collagen, type VIII, alpha 2 | Extracellular Space | other | 4.876128 | -1.41058 |

(continued)

| ID | Symbol | Entrez Gene Name | Location | Type(s) | q value | Fold change |
|---|---|---|---|---|---|---|
| FJX1 | FJX1 | four jointed box 1 (Drosophila) | Extracellular Space | other | 0.301273 | -2.32761 |
| TAC1 | TAC1 | tachykinin, precursor 1 | Extracellular Space | other | 9.443493 | -1.26207 |
| THBS1 | THBS1 | thrombospondin 1 | Extracellular Space | other | 9.155824 | -1.35908 |

**Table 2 Up-regulated genes**

| ID | Symbol | Entrez Gene Name | Location | Type(s) | q value | Fold change |
|---|---|---|---|---|---|---|
| ACPP | ACPP | acid phosphatase, prostate | Extracellular Space | phosphatase | 9.500153 | 1.769193 |
| ACSS1 | ACSS1 | acyl-CoA synthetase short-chain family member 1 | Cytoplasm | enzyme | 3.617024 | 1.211557 |
| ADAMTS 15 | ADAMTS 15 | ADAM metallopeptidase with thrombospondin type 1 motif, 15 | Extracellular Space | peptidas e | 8.787849 | 1.094887 |
| ADH6 | ADH6 | alcohol dehydrogenase 6 (class V) | Cytoplasm | enzyme | 3.617024 | 1.176887 |
| ADSSL1 | ADSSL1 | adenylosuccinate synthase like 1 | Cytoplasm | enzyme | 7.885502 | 0.854385 |
| ALDH1A 3 | ALDH1A 3 | aldehyde dehydrogenase 1 family, member A3 | Cytoplasm | enzyme | 3.196117 | 1.792436 |
| AMY1 C | AMY1A (includes others) | amylase, alpha 1A (salivary) | Extracellular Space | enzyme | 7.333469 | 0.899842 |
| B3GALT 4 | B3GALT 4 | UDP-Gal:betaGlcNAc beta 1,3-galactosyltransferase, polypeptide 4 | Cytoplasm | enzyme | 3.143549 | 1.174816 |
| B4GALT 6 | B4GALT 6 | UDP-GaL:betaGlcNAc beta 1,4-galactosyltransferase, polypeptide 6 | Cytoplasm | enzyme | 2.488273 | 1.070201 |
| BMP7 | BMP7 | bone morphogenetic protein 7 | Extracellular Space | growth factor | 5.211284 | 1.122622 |
| CA6 | CA6 | carbonic anhydrase VI | Extracellular Space | enzyme | 3.617024 | 1.029014 |
| CCL1 | CCL1 | chemokine (C-C motif) ligand 1 | Extracellular Space | cytokine | 9.762848 | 1.135392 |
| CCL26 | CCL26 | chemokine (C-C motif) ligand 26 | Extracellular Space | cvtokine | 1.447061 | 1.66153 |
| CTSF | CTSF | cathepsin F | Cytoplasm | peptidas e | 0.393105 | 1.610012 |
| CYP3A5 | CYP3A5 | cytochrome P450, family 3, subfamily A, polypeptide 5 | Cytoplasm | enzyme | 1.312691 | 1.213781 |

(continued)

| ID | Symbol | Entrez Gene Name | Location | Type(s) | q value | Fold change |
|---|---|---|---|---|---|---|
| CYP4F3 | CYP4F3 | cytochrome P450, family 4, subfamily F, polypeptide 3 | Cytoplasm | enzyme | 1.145026 | 1.394588 |
| CYP4V2 | CYP4V2 | cytochrome P450, family 4, subfamily V, polypeptide 2 | Cytoplasm | enzyme | 9.544845 | 0.82527 |
| CYP4X1 | CYP4X1 | cytochrome P450, family 4, subfamily X, polypeptide 1 | Cytoplasm | enzyme | 7.780925 | 1.061522 |
| DCLK1 | DCLK1 | doublecortin-like kinase 1 | Cytoplasm | kinase | 0.990043 | 1.436873 |
| DHRS3 | DHRS3 | dehydrogenase/reductas e (SDR family) member 3 | Cytoplasm | enzyme | 9.500153 | 0.820622 |
| DTD1 | DTD1 | D-tyrosyl-tRNA deacylase 1 homolog (S. cerevisiae) | Cytoplasm | enzyme | 0.486825 | 1.511435 |
| DUSP13 | DUSP13 | dual specificity phosphatase 13 | Cytoplasm | phosphat ase | 6.810082 | 1.045486 |
| DZIP3 | DZIP3 | DAZ interacting protein 3, zinc finger | Cytoplasm | enzyme | 9.500153 | 0.853284 |
| F9 | F9 | coagulation factor IX | Extracellular Space | peptidas e | 5.835312 | 1.604713 |
| GBP3 | GBP3 | guanylate binding protein 3 | Cytoplasm | enzyme | 8.493268 | 0.846187 |
| GDA | GDA | guanine deaminase | Cytoplasm | enzyme | 6.889679 | 1.596347 |
| GNAL | GNAL | guanine nucleotide binding protein (G protein), alpha activating activity polypeptide, olfactory type | Cytoplasm | enzyme | 6.889679 | 0.995972 |
| GPX6 | GPX6 | glutathione peroxidase 6 (olfactory) | Extracellular Space | enzyme | 6.056672 | 1.701714 |
| HSD17B 13 | HSD17B 13 | hydroxysteroid (17-beta) dehydrogenase 13 | Extracellular Space | enzyme | 2.797309 | 1.509712 |
| IGBP1 | IGBP1 | immunoglobulin (CD79A) binding protein 1 | Cytoplasm | phosphat ase | 9.347548 | 0.803832 |
| IGF2 | IGF2 | insulin-like growth factor 2 (somatomedin A) | Extracellular Space | growth factor | 3.928841 | 1.184617 |
| IL1B | IL1B | interleukin 1, beta | Extracellular Space | cytokine | 5.211284 | 1.135797 |
| IL1RN | IL1RN | interleukin 1 receptor antagonist | Extracellular Space | cytokine | 4.588739 | 1.004466 |
| INPP5K | INPP5K | inositol polyphosphate-5-phosphatase K | Cytoplasm | phosphat ase | 9.544845 | 0.775302 |
| JAK1 | JAK1 | Janus kinase 1 | Cytoplasm | kinase | 6.056672 | 0.872602 |
| LEFTY1 | LEFTY1 | left-right determination factor 1 | Extracellular Space | growth factor | 4.653116 | 1.067174 |
| LEP | LEP | leptin | Extracellular Space | growth factor | 9.544845 | 1.280507 |

(continued)

| ID | Symbol | Entrez Gene Name | Location | Type(s) | q value | Fold change |
|---|---|---|---|---|---|---|
| LIPF | LIPF | lipase, gastric | Extracellular Space | en7yme | 1.821924 | 1.348977 |
| LOXL4 | LOXL4 | lysyl oxidase-like 4 | Extracellular Space | enzyme | 6.056672 | 0.867586 |
| MARK1 | MARK1 | MAP/microtubule affinity-regulating kinase 1 | Cytoplasm | kinase | 3.216837 | 1.450886 |
| MMP12 | MMP12 | matrix metallopeptidase 12 (macrophage elastase) | Extracellular Space | peptidase | 0.490071 | 1.631485 |
| MMP16 | MMP16 | matrix metallopeptidase 16 (membrane-inserted) | Extracellular Space | peptidase | 4.168656 | 1.799388 |
| MMP9 | MMP9 | matrix metallopeptidase 9 (gelatinase B, 92kDa gelatinase, 92kDa type IV collagenase) | Extracellular Space | peptidase | 4.588739 | 1.152273 |
| MPP4 | MPP4 | membrane protein, palmitoylated 4 (MAGUK p55 subfamily member 4) | Cytoplasm | kinase | 7.768987 | 0.86115 |
| MTHFR | MTHFR | methylenetetrahydrofolat e reductase (NAD(P)H) | Cytoplasm | enzyme | 9.762848 | 0.815308 |
| OGN | OGN | osteoglycin | Extracellular Space | growth factor | 0.440183 | 1.567356 |
| PAOX | PAOX | polyamine oxidase (exo-N4-amino) | Cytoplasm | enzyme | 9.762848 | 0.857204 |
| PDE1A | PDE1A | phosphodiesterase 1 A, calmodulin-dependent | Cytoplasm | enzyme | 1.709551 | 1.310078 |
| PDE4C | PDE4C | phosphodiesterase 4C, cAMP-specific | Cytoplasm | enzyme | 7.539559 | 1.01713 |
| PDK4 | PDK4 | pyruvate dehydrogenase kinase, isozyme 4 | Cytoplasm | kinase | 8.108911 | 0.818656 |
| PIM1 | PIM1 | pim-1 oncogene | Cytoplasm | kinase | 3.346465 | 1.101022 |
| PITPNM2 | PITPNM 2 | phosphatidylinositol transfer protein, membrane-associated 2 | Cytoplasm | enzyme | 6.334792 | 0.870237 |
| PLAT | PLAT | plasminogen activator, tissue | Extracellular Space | peptidase | 6.535021 | 0.88969 |
| PLB1 | PLB1 | phospholipase B1 | Cytoplasm | enzyme | 1.206892 | 1.566276 |
| PLD1 | PLD1 | phospholipase D1, phosphatidylcholine-specific | Cytoplasm | enzyme | 8.787849 | 0.93338 |
| PLGLB1 | PLGLB1/ PLGLB2 | plasminogen-like B2 | Extracellular Space | peptidase | 7.333469 | 0.944078 |
| PON3 | PON3 | paraoxonase 3 | Extracellular Space | enzyme | 4.588739 | 1.01348 |
| PRODH2 | PRODH2 | proline dehydrogenase (oxidase) 2 | Cytoplasm | enzyme | 0.980142 | 1.716334 |

(continued)

| ID | Symbol | Entrez Gene Name | Location | Type(s) | q value | Fold change |
|---|---|---|---|---|---|---|
| PRSS23 | PRSS23 | protease, serine, 23 | Extracellular Space | peptidase | 5.835312 | 1.012222 |
| RAB37 | RAB37 | RAB37, member RAS oncogene family | Cytoplasm | enzyme | 6.334792 | 0.905196 |
| RAB39 | RAB39 | RAB39, member RAS oncogene family | Cytoplasm | enzyme | 9.155824 | 0.809517 |
| RND3 | RND3 | Rho family GTPase 3 | Cytoplasm | enzyme | 9.500153 | 0.912389 |
| RPS6KB 1 | RPS6KB 1 | ribosomal protein S6 kinase, 70kDa, polypeptide 1 | Cytoplasm | kinase | 2.739944 | 1.480561 |
| SPP1 | SPP1 | secreted phosphoprotein 1 | Extracellular Space | cytokine | 2.739944 | 1.471148 |
| SPRED2 | SPRED2 | sprouty-related, EVH1 domain containing 2 | Extracellular Space | cytokine | 9.544845 | 0.825032 |
| ST3GAL 6 | ST3GAL 6 | ST3 beta-galactoside alpha-2,3-sialyltransferase 6 | Cytoplasm | enzyme | 1.918337 | 1.174742 |
| STK31 | STK31 | serine/threonine kinase 31 | Cytoplasm | kinase | 6.810082 | 1.09185 |
| STS | STS | steroid sulfatase (microsomal), isozyme S | Cytoplasm | enzyme | 9.762848 | 0.867689 |
| TDH | TDH | L-threonine dehydrogenase | Cytoplasm | enzyme | 4.876127 | 1.414347 |
| TRIB1 | TRIB1 | tribbles homolog 1 (Drosophila) | Cytoplasm | kinase | 3.238106 | 1.07006 |
| ULK2 | ULK2 | unc-51-like kinase 2 (C. elegans) | Cytoplasm | kinase | 6.535021 | 1.068956 |
| WWP2 | WWP2 | WW domain containing E3 ubiquitin protein ligase 2 | Cytoplasm | enzyme | 8.787849 | 0.910502 |
| ZADH2 | ZADH2 | zinc binding alcohol dehydrogenase domain containing 2 | Cytoplasm | enzyme | 5.211284 | 1.004706 |

The invention will be further understood with reference to the following non-limiting examples.

## EXAMPLES

### Example 1. Gene expression microarray analysis of lymphocytes treated with rapamycin

Two-colour microarray based gene expression analysis (Agilent Technologies) was used to identify genes that were differentially expressed in lymphocytes treated with rapamycin compared to lymphocytes that were left untreated. The microarray used was the Agilent Whole Human Genome Microarray: 4x44K.

*1.1 Lymphocyte culture with and without rapamycin*

Two parallel lymphocyte cultures were set up in RPMI medium supplemented with 10% FCS at a concentration of $1 \times 10^6$ cells per 1 ml of culture media. Phytohaemaglutinin (PHA) was added to the cultures at a final concentration of 22 μg/ml to activate the lymphocytes. Cultures were incubated for 48 hours at 37°C in a humidified atmosphere containing 5% $CO_2$. After 48 hours culture, one culture was treated with 100 ng/ml rapamycin, while the other untreated culture was kept as a control. After a further 24 hours, the cells of each lymphocyte culture were harvested by centrifugation.

*1.2 RNA extraction*

RNA was extracted from the lymphocyte pellets using TRI Reagent<sup>®</sup> in accordance with standard protocol. To ensure that there was no DNA contamination, the RNA was treated with DNase (Qiagen) according to the manufacturer's standard protocol. RNA quality was determined by RNA nano-chip analysis using an Agilent 2100 Bioanalyser, also according to standard protocol. The RNA quality of the samples ranged from 8-10 RNA Integrity Number (RIN), indicative of good quality RNA suitable for gene expression microarray.

*1.3 Conversion of RNA to cDNA*

200 ng of RNA was converted to cDNA with the low-input quick amplification labelling kit (Agilent, 5190-2305). Spike Mix A and B were prepared separately. For each, the spike mix was thawed and vortexed, incubated at 37°C for 5 minutes, and diluted in the provided dilution buffer (see Table 3 for dilution protocol). 2 $\mu$l of diluted spike mix A and spike mix B were added to 200 ng of RNA in a 1.5 $\mu$l volume from the rapamycin-treated and untreated lymphocytes respectively. The T7 promoter primer was prepared as shown in **Table** 4, and 1.8 $\mu$l added per 3.5 $\mu$l sample. The samples were incubated at 65°C for 10 minutes, and placed on ice for 5 minutes. The cDNA master mix was set up immediately prior to use as shown in Table 5. 4.7 $\mu$l of the cDNA master mix was added per sample, and samples incubated at 40°C for 2 hours; 70°C for 15 minutes; and placed on ice for 5 minutes.

**Table 3 Preparation of Spike Mix A and Spike Mix B**

| Starting amount of RNA | | Serial dilutions | | | |
|---|---|---|---|---|---|
| Total RNA (nq) | Concentration (ng/$\mu$l) | First | Second | Third | Spike mix volume per labelling ($\mu$l) |
| **200** | **133.3** | **1:20** | **1:40** | **1:16** | **2** |

**Table 4 Dilution of T7 promoter primer**

| Component (Agilent) | Volume ($\mu$l) per reaction |
|---|---|
| T7 promoter primer | 0.8 |
| Nuclease free water | 1 |
| **Total volume** | **1.8** |

**Table 5 cDNA master mix**

| Components (Agilent) | Volume ($\mu$l) per reaction |
|---|---|
| 5x first strand buffer (pre-warmed at 80°C for 4 minutes prior to use) | 2 |
| 0.1 M DTT | 1 |
| 10mM dNTP mix | 0.5 |
| AffinityScript RNase Block mix | 1.2 |
| **Total volume** | **4.7** |

*1.4 Conversion of cDNA to labelled cRNA*

The low-input quick amplification labelling kit (Agilent, 5190-2305) was used to convert the cDNA to labelled cRNA. The transcription master mix was set up immediately prior to use as shown in Table 6. Spike Mix A samples were added to transcription master mix prepared with Cyanine 3-CTP (green), whereas Spike Mix B samples were added to transcription master mix prepared with Cyanine 5-CTP (red). 6$\mu$l of the transcription master mix was added per 10$\mu$l cDNA sample, and incubated at 40°C for 2 hours.

**Table 6 Transcription master mix**

| Component (Agilent) | Volume (μl) per reaction |
|---|---|
| Nuclease free water | 0.75 |
| 5 x transcription buffer | 3.2 |
| 0.1 M DTT | 0.6 |
| NTP mix | 1 |
| T7 RNA polymerase Blend (store on ice until use) | 0.21 |
| Cyanine 3-CTP or Cyanine 5-CTP | 0.24 |
| **Total volume** | 6 |
| *1.5 cRNA purification and quantification* | |

The cRNA was purified by standard RNeasy mini column protocol (Qiagen). The cRNA was quantified with a nanodrop spectrophotometer on the Microarray setting. The yield and specific activity of the cRNA samples were calculated as follows:

$$\text{Yield} = \text{Concentration of cRNA x remaining cRNA volume} / 1000$$

$$\text{Specific activity} = \text{Concentration of Cy3 or Cy5} / \text{Concentration of cRNA x 1000}$$

The specific activity and yield of all the samples were greater than 6 and 825 ng respectively indicating suitability for hybridisation.

*1.6 Hybridisation*

825 ng of labelled cRNA was added to various fragmentation components as described in Table 7, incubated at 60ºC for 30 minutes, and cooled on ice for 1 minute. 55 μl of GEx Hybridisation buffer HI-RPM was subsequently added to stop fragmentation. The samples were mixed, centrifuged at 13,000 rotations per minute (RPM) for 1 minute, and placed on ice in preparation for microarray assembly. The microarray of choice was the 4x44K Agilent Whole Human Genome Microarray. A clean gasket slide was placed into the chamber base, 100μl of sample dispensed per well, and array placed onto the gasket slide to facilitate hybridisation. The gasket and microarray slides were clamped together and incubated at 65ºC for 17 hours.

**Table 7 Fragmentation components**

| Components (Agilent) | Volume/ Mass (for 4x44K microarray) |
|---|---|
| Cy3-labelled cRNA | 825ng |
| Cy5-labelled cRNA | 825ng |
| 10 x blocking agent | 11 μl |
| Nuclease free water | Bring total volume to 52.8 μl |
| 25 x fragmentation buffer | 2.2 μl |
| **Total volume** | **55 μl** |

Following hybridisation, the microarrays were washed in various wash solutions as described in Table 8. To summarise, the microarrays were removed from the hybridisation assembly and carefully separated from the gasket slide whilst fully submersed in wash buffer 1. Slides were washed for 1 minute, transferred to wash buffer 2 at 3ºC for 1 minute, and washed in acetonitrile for 10 seconds. The final wash was in stabilisation and drying solution for 30 seconds. The slides were air-dried and stored in a dark slide box in a nitrogen rich environment prior to scanning on the Agilent C Scanner. The AgilentHD_GX_2color programme was used, and settings amended as shown in Table 9.

**Table 8 Microarray wash protocol**

| Dish | Wash buffer (Agilent) | Temp | Time |
|---|---|---|---|
| 1 | GE Wash buffer 1 | Room temperature (RT) | |
| 2 | GE Wash buffer 1 | RT (with stir bar) | 1 min |
| 3 | GE Wash buffer 2 | 37°C (with stir bar) | 1 min |
| 4 | Acetonitrile | RT (with stir bar) | 10 sec |
| 5 | Stabilisation and drying solution (Agilent) | RT (with stir bar) | 30 sec |

**Table 9 Microarray scanner settings for two-colour microarray based gene expression analysis (Agilent)**

| | 4x44K HD microarray format |
|---|---|
| **Dye Channel** | Red and Green |
| **Scan region** | Scan area (61 x 21.6mm) |
| **Scan resolution ($\mu$M)** | 5 |
| **Tiff** | 20 bit |

*1.7 Data analysis*

The feature extraction programme was used to collate the Microarray layout with the output of the scanner. The output was normalised to various housekeeping genes present on the microarray, and results processed to provide a list of genes that were differentially expressed in rapamycin-treated lymphocytes compared to untreated lymphocytes. Estimates of the false discovery rate (FDR) were provided for each gene. For the purpose of our study, genes with a FDR up to 10% were accepted as differentially expressed. Genes determined to be down-regulated and up-regulated in response to rapamycin are shown in Tables 1 and 2, respectively.

**Example 2. Expression analysis of rapamycin-sensitive genes in lymphocytes isolated from human subjects**

Lymphocytes are isolated from venous blood samples collected from the human subjects for testing. The venous blood sample is collected from the patient in a heparin vacutainer and transported into the laboratory at room temperature within 48 hours. The lymphocytes are separated from the blood according to standard protocols using Lymphoprep, Histopaque, Ficoll or an equivalent standard reagent.

Alternatively, the venous blood is collected directly into a BD Vacutainer® CPT™ cell preparation tube with sodium heparin and lymphocyte separation is carried out, using one of the techniques described above, instantly. The lymphocyte sample is then transported to the laboratory for analysis.

For each lymphocyte sample taken from a human subject, two parallel lymphocyte cultures are set up in RPMI medium supplemented with 10% FCS at a concentration of $1 \times 10^6$ cells per 1 ml of culture media. Phytohaemaglutinin (PHA) is added to the cultures at a final concentration of 22 $\mu$g/ml to activate the lymphocytes. Cultures are incubated for 48 hours at 37 °C in a humidified atmosphere containing 5% $CO_2$. After 48 hours culture, one culture is treated with 100ng/ml rapamycin, while the other untreated culture is kept as a control. After a further 24 hours, the cells of each lymphocyte culture are frozen to -80 °C and stored at this temperature until further analysis.

Analysis of gene expression is carried out by analysing the levels of corresponding protein for the target genes of interest. Protein levels are determined in accordance with standard protocols, such as ELISAs carried out in accordance with manufacturer's instructions.

**Example 3 Differential gene expression responses to rapamycin measured in Alzheimer's disease patients**

3.1 Blood collection and lymphocyte separation

Peripheral blood samples from elderly subjects were provided by the Oxford Project to Investigate Memory and Ageing (OPTIMA) subject to ethical approval and informed patient and carer consent. The samples provided were from Alzheimer patients who fulfilled the NINCDS-ARDRA criteria for probable AD (n=24) or from healthy age-matched controls (n=21). Appropriate consent and ethical approval was sought before the study began.

Peripheral blood was collected in Heparin vacutainers and shipped at room temperature. The separation of lymphocytes was carried out within 24 hours of blood collection using established protocols [Lymphoprep]. Briefly the blood was diluted 1:1 with PBS (Ca and Mg free, Sigma). Ten ml diluted blood was carefully layered onto 4 ml Lymphoprep (Axis Shield UK) in 15-ml conical centrifuge tubes. Samples were centrifuged at 800 x g for exactly 30 minutes at room temperature. The upper layer (within 0.5 cm of the opaque interface) was discarded. The opaque interface containing mononuclear cells was carefully removed into a clean 15 ml conical centrifuge tube containing PBS (Ca and Mg free, Sigma) and mixed by turning the tube a few times gently. The cells were centrifuged at 400 x g for 10 minutes at room temperature. The supernatant was aspirated to approximately 1 cm from the pellet and discarded. To this tube was added 2 ml of Isotonic PBS (Ca and Mg free, Sigma) and the pellet was resuspended by triturating with a pastette (3 aspirations), then made up to a volume of 14 ml and mixed by turning the tube a few times gently. The cell suspension was centrifuged at 400 x g for 10 minutes at room temperature. The supernatant was aspirated and discarded. The cell pellet was resuspended with 9:1 FCS:DMSO and stored at -70 °C (for at least 24 hours) before placing into liquid nitrogen.

3.2 Treatment of lymphocytes with rapamycin

Frozen lymphocytes were thawed in batches and washed in RPMI-1640 to remove freezing medium. The cells were then cultured (1 million/ml density) in RPMI-1640 medium supplemented with L-glutamine (2%), Penicillin/Streptomycin (1%), PHA (2.5% of PAA PHA) and Foetal calf serum (15%) for 48 hours.

From each patient two sets of identical cultures were set up in 96 well plates (6 wells in each set). Following the initial 48 hours culture, half of the cultures from each set were treated with Rapamycin (100ng/ml) while half were treated with DMSO (solvent for Rapamycin) alone for a further 24 hours. At the end of the treatment period (total 72 hours in culture), one set of cultures was collected for LDH assay by freezing the samples at -200 °C. The second set of cultures was collected for ELISA assay by freezing the samples at -70 °C

3.3 Gene expression analysis by ELISA

The expression of interleukin 1 beta (IL1B), interleukin 2 (IL-2), interleukin 6 (IL-6) and interleukin 10 (IL-10) was measured at the protein level by ELISA using standard kits as follows:

    Human IL-1 beta Ready-SET-Go 10 plate kit (eBiosciences, 88-7010-86)
    Human IL-6 Ready-SET-Go 10 plate kit (eBiosciences, 88-7066-86)
    Human IL-10 Ready-SET-Go 10 plate kit (eBiosciences, 88-7106-86)
    Human IL-2 Screening Set (Thermo-Fisher, ESS0010)

The kits are provided with appropriate capture antibodies, recombinant proteins to be used as standards, biotin detection antibodies, avidin-HRP, assay diluent, TMB solution and coating buffer.

Serial dilutions of the human recombinant IL-1 beta, IL-2, IL-6 and IL-10 proteins were prepared for use in generating standard calibration curves. The top standard concentration for each ELISA was as follows:

| IL-1 beta | 500 pg/ml |
|---|---|
| IL-6 | 200 pg/ml |
| IL-10 | 300 pg/ml |
| IL-2 | 1500 pg/ml |

Two-fold serial dilutions of the recombinant protein preparations were prepared in the same medium as that of the samples (RPMI) and seven standards of decreasing concentration were prepared (1/1, 1/2, 1/4, 1/8, 1/16, 1/32, 1/64, 0).

The ELISA was performed as follows:-

    A high-bonding 96-well plate was coated with 100 $\mu$L/well of coating antibody (1000 $\mu$g/mL in 1X assay diluent) and the plate was sealed and incubated overnight (IL-1 beta and IL-6) or over two nights (IL-10 and IL-2) at 4°C.

Any unbound coating antibody was removed from the wells of the plate by aspirating and washing the wells with a ELx405 plate washer (programme 13 (E1); 300 $\mu$L/well wash solution and 1 min soaking between 5 wash steps). Excess wash solution was removed from the wells by blotting with absorbent paper.

The coated wells were blocked with 300 $\mu$L/%well 1X assay diluent. After addition of the blocking solution, the plates were sealed and incubated at room temperature for 1 hour.

The plates containing the samples described in section 3.2 above were removed from the -80°C freezer. They were allowed to thaw completely and were then spun briefly to remove any condensation from the lid. The samples were mixed by pipetting up and down gently several times with a multichannel pipette, using clean tips for each column.

The liquid was removed from the wells of the blocked ELISA plate by aspirating and the wells were washed with the ELx405 plate washer (programme 13). Any excess wash solution was removed with a clean sheet of absorbent paper.

The calibration curve was set up by adding 100 $\mu$L of each concentration of protein standard to column 1 of the ELISA plate. Using a multichannel pipette and clean tips, 100 $\mu$L of each sample was distributed into columns 2-12 of the ELISA plate. The plate was sealed and incubated overnight at 4 °C.

The sample-containing ELISA plate was removed from 4°C, and the wells were aspirated and washed with the ELx405 plate washer (programme 13). Any excess wash solution was removed with a clean sheet of absorbent paper.

100 $\mu$L of the detection antibody (250 ng/mL in 1X assay diluent) was added to each well and the plate was sealed and incubated at room temperature for 1 hour. The liquid was removed from the wells by aspirating and the wells were washed with the ELx405 plate washer (programme 13). Any excess wash solution was removed with a clean sheet of absorbent paper.

100 $\mu$L of 1X Streptavidin-HRP was added to each well, the plates were covered with foil and incubated at room temperature for 30 min. The liquid was removed from the wells by aspirating and the wells were washed with the ELx405 plate washer (programme 13). Any excess wash solution was removed with a clean sheet of absorbent paper.

100 $\mu$L of 1X substrate was added to each well, the plates were covered with foil and incubated at room temperature for 15 min. The reaction was stopped by addition of 100 $\mu$L of 1X stop solution or 50 $\mu$L 2N $H_2SO_4$ to each well. Adsorption values were read at 450 nm.

3.4 Results

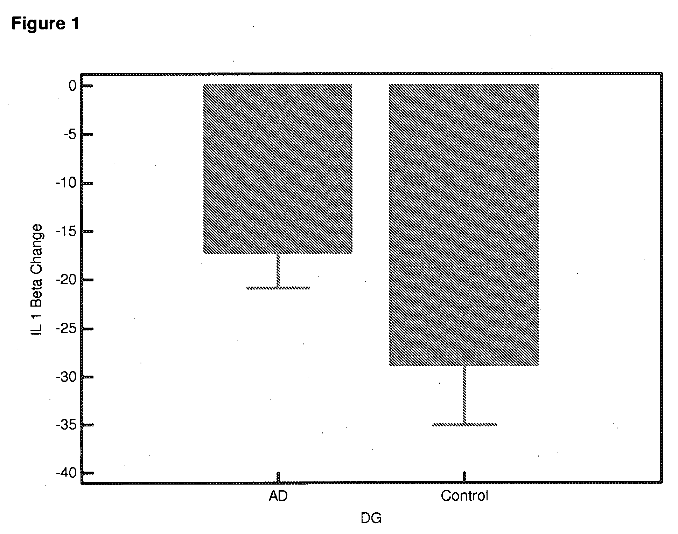

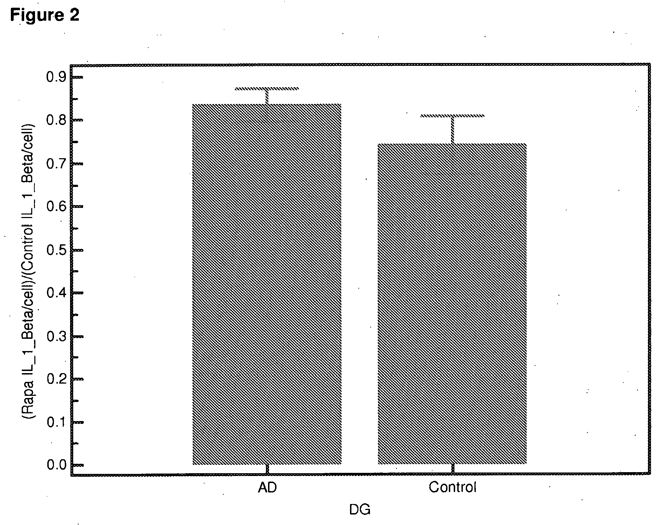

The reduction of total IL 1 beta levels in dividing lymphocytes in response to Rapamycin was higher in control subjects than in AD patients, as shown Figure 1. The reduction of IL 1 beta/live cell in dividing lymphocytes in response to Rapamycin was also more significant in control subjects than in AD patients, as shown in Figure 2.

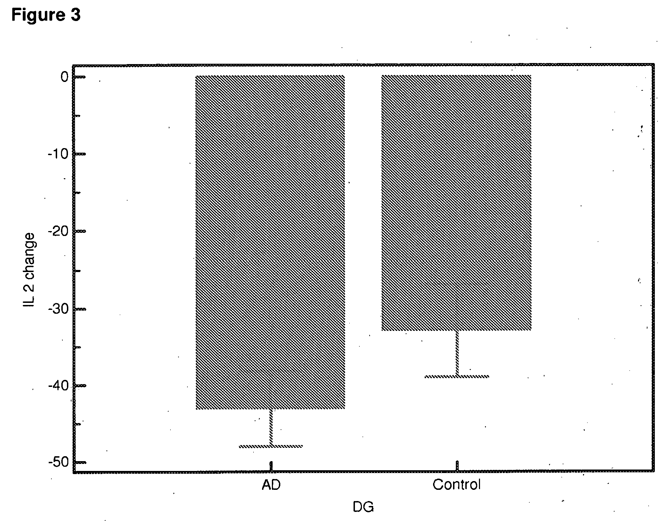

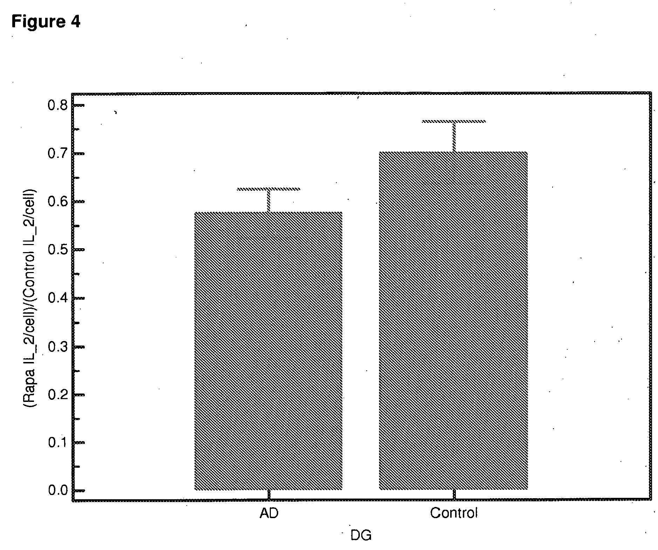

The reduction of IL 2 levels in dividing lymphocytes in response to Rapamycin was lower in control subjects than in AD patients, as shown in Figure 3. The reduction of IL 2/live cell in dividing lymphocytes in response to Rapamycin was also less significant in Control subjects than in AD patients, as shown in Figure 4.

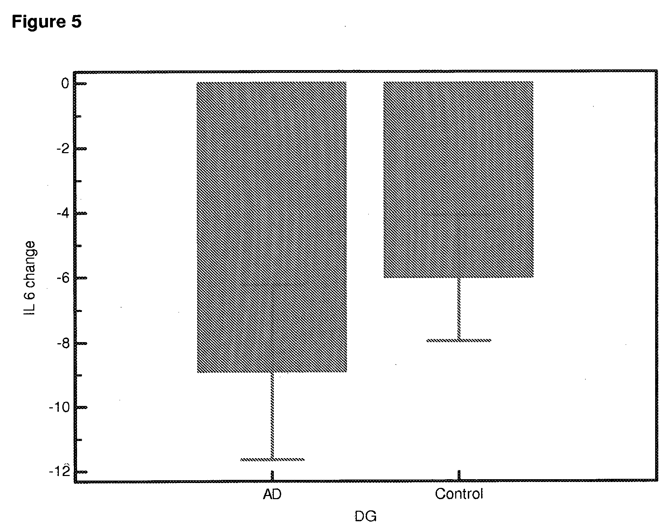

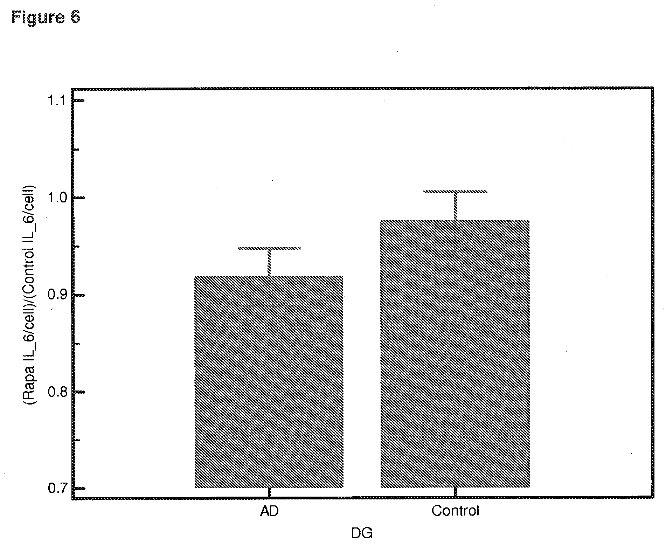

The reduction of IL 6 levels in dividing lymphocytes in response to Rapamycin was lower in Control subjects than in AD patients, as shown in Figure 5. The reduction of IL 6 levels/cell in dividing lymphocytes in response to Rapamycin was also less significant in Control subjects than in AD patients, as shown in Figure 6.

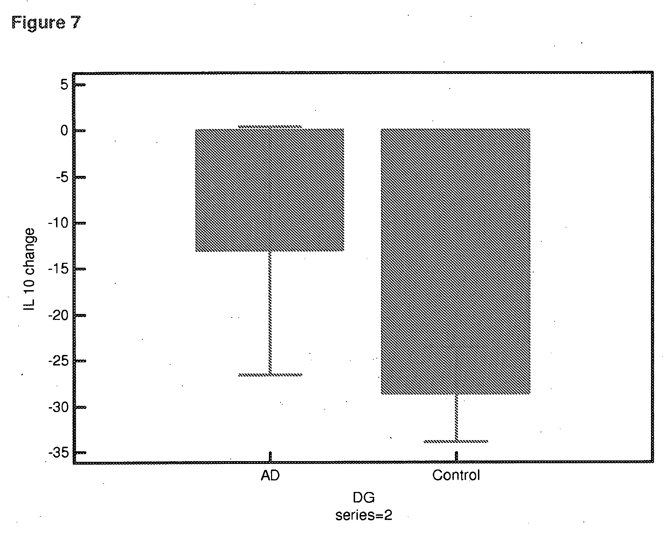

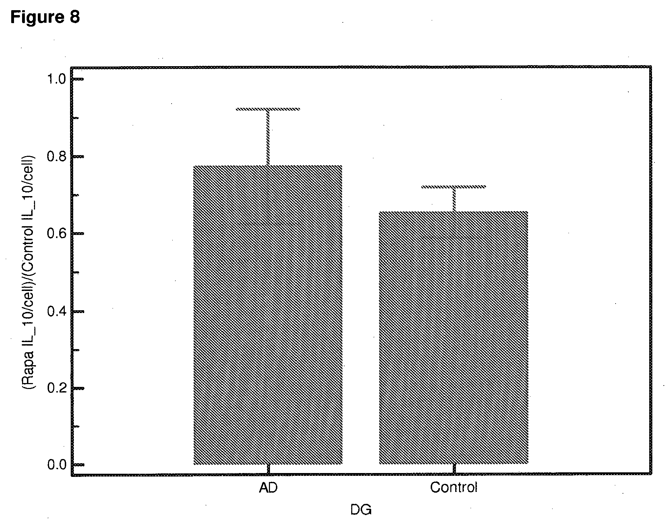

The reduction of IL 10 levels in dividing lymphocytes in response to Rapamycin was higher in Control subjects than in AD patients, as shown in Figure 7. The reduction of IL 10 levels/cell in dividing lymphocytes in response to Rapamycin was more pronounced in Control subjects than in AD patients, as shown in Figure 8.

3.4.1 Logistic regression and ROC curve analysis for IL 1 beta

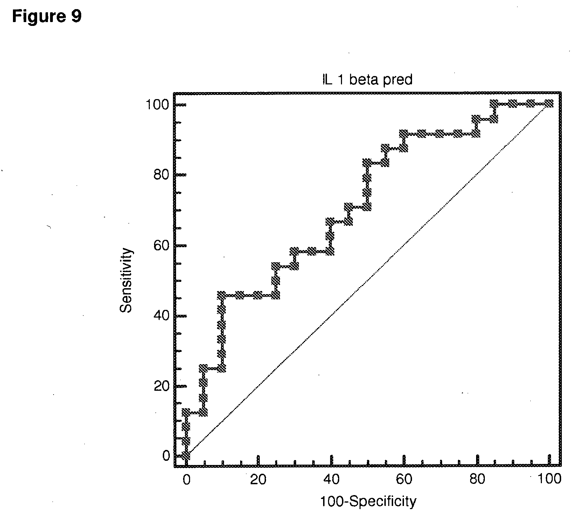

Logistic regression and ROC curve analysis indicates that the combination of change in total IL 1 beta levels and IL1 beta levels/cell in response to Rapamycin could alone distinguish AD patients from controls (see Figure 9 and analysis below).

**Logistic regression**

| Dependent Y | DG |
|---|---|
| Select | |

**OVERALL MODEL FIT**

| Null model - 2 Log Likelihood | 60.633 |
|---|---|
| Full model -2 Log Likelihood | 53.669 |
| Chi-square | 6.963 |

(continued)

| DF | 2 |
|---|---|
| Significance level | P = 0.0308 |

**COEFFICIENTS AND STANDARD ERRORS**

| Variable | Coefficient | Std. Error | P |
|---|---|---|---|
| IL_1_Beta_Change | 0.22372 | 0.10226 | 0.0287 |
| (Rapa IL_1_Beta/cell)/(Control IL_1_Beta_C/cell) | -19.79583 | 9.31493 | 0.0336 |
| Constant | 20.8900 | | |

**ROC CURVE ANALYSIS**

| Area under the ROC curve (AUC) | 0.708 |
|---|---|
| Standard Error | 0.0784 |
| 95% Confidence interval | 0.552 to 0.835 |

**ROC curve**

| Variable | IL_1_beta_pred IL 1 beta pred |
|---|---|
| Classification variable | DG |

**AREA UNDER THE ROC CURVE (AUC)**

| Area under the ROC curve (AUC) | 0.708 |
|---|---|
| Standard Error[a] | 0.0784 |
| 95% Confidence interval[b] | 0.555 to 0.862 |
| z statistic | 2.658 |
| Significance level P (Area=0.5) | 0.0079 |
| [a] Hanley & McNeil, 1982 [b] AUC $\pm$ 1.96 SE | |

**YOUDEN INDEX**

| Youden index J | 0.3583 |
|---|---|
| Associated criterion | >0.6789 |

3.4.2 Logistic regression and ROC curve analysis for IL-2

Logistic regression and ROC curve analysis indicates that changes in IL-2 expression by rapamycin could alone distinguish AD patients from controls (see analysis below).

**Logistic regression**

| Dependent Y | DG |
|---|---|
| Select | |

**OVERALL MODEL FIT**

| Null model - 2 Log Likelihood | 62.183 |
|---|---|
| Full model -2 Log Likelihood | 55.823 |
| Chi-square | 6.360 |
| DF | 2 |
| Significance level | P = 0.0416 |

**ODDS RATIOS AND 95% CONFIDENCE INTERVALS**

| Variable | Odds ratio | 95% CI |
|---|---|---|
| IL_2_change | 1.2021 | 0.9860 to 1.4656 |
| (Rapa IL_2/cell)/(Control IL_2/cell) | 0.0000 | 0.0000 to 3.9337 |

**ROC CURVE ANALYSIS**

| Area under the ROC curve (AUC) | 0.679 |
|---|---|
| Standard Error | 0.0793 |
| 95% Confidence interval | 0.523 to 0.810 |

**ROC curve**

| Variable | IL_2_pred IL 2 pred |
|---|---|
| Classification variable | DG |
| Select | |

**AREA UNDER THE ROC CURVE (AUC)**

| Area under the ROC curve (AUC) | 0.679 |
|---|---|
| Standard Error[a] | 0.0801 |
| 95% Confidence interval[b] | 0.522 to 0.836 |
| z statistic | 2.228 |
| Significance level P (Area=0.5) | 0.0258 |
| [a] DeLong et al., 1988 [b] AUC $\pm$ 1.96 SE | |

**YOUDEN INDEX**

| Youden index J | 0.3095 |
|---|---|

(continued)

| Associated criterion | >0.6392 |
|---|---|

### 3.4.3 Logistic regression and ROC curve analysis for IL 1 beta in combination with IL-6

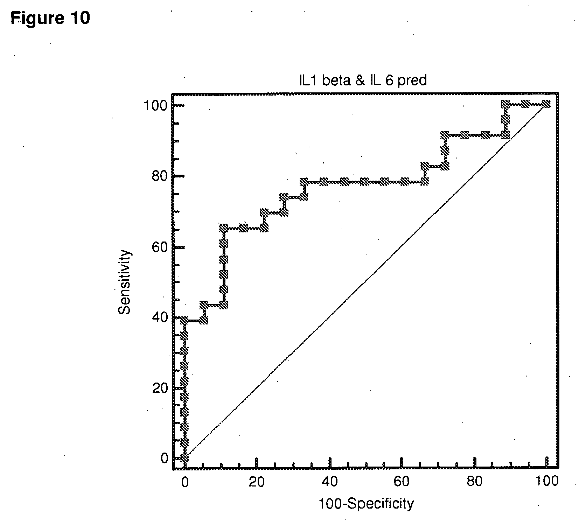

The combination of IL 6 changes with the IL 1 beta changes allowed a correct patient diagnosis from the ELISA results (see Figure 10 and analysis below).

**Logistic regression**

| Dependent Y | DG |
|---|---|
| Select | |

**OVERALL MODEL FIT**

| Null model - 2 Log Likelihood | 56.227 |
|---|---|
| Full model -2 Log Likelihood | 45.672 |
| Chi-square | 10.555 |
| DF | 5 |
| Significance level | P = 0.1032 |

**COEFFICIENTS AND STANDARD ERRORS**

| Variable | Coefficient | Std. Error | P |
|---|---|---|---|
| (Rapa IL_1_Beta/cell)/(Control | -94.36426 | 54.44730 | 0.0831 |
| IL_1_Beta_C/cell) | | | |
| (Rapa IL_6/cell)/(Control IL_6_C/cell) | 187.20670 | 98.82951 | 0.0582 |
| IL_1_Beta_Change | 1.01041 | 0.57969 | 0.0813 |
| IL_6_change | -1.89564 | 1.00576 | 0.0595 |
| % change in cell numbers in response to Rapamycin | -1.23152 | 0.75159 | 0.1013 |
| Constant | -86.4594 | | |

**ROC CURVE ANALYSIS**

| Area under the ROC curve (AUC) | 0.768 |
|---|---|
| Standard Error | 0.0748 |
| 95% Confidence interval | 0.610 to 0.885 |

**ROC curve**

| variable | 1L1_beta_&_IL_6_pred IL1 beta 8 IL 6 pred |
|---|---|
| Classification variable | DG |

**AREA UNDER THE ROC CURVE (AUC)**

| | |
|---|---|
| Area under the ROC curve (AUC) | 0.768 |
| Standard Error[a] | 0.0748 |
| 95% Confidence interval[b] | 0.621 to 0.915 |
| z statistic | 3.582 |
| Significance level P (Area=0.5) | 0.0003 |
| [a] Hanley & McNeil, 1982 [b] AUC $\pm$ 1.96 SE | |

**YOUDEN INDEX**

| | |
|---|---|
| Youden index J | 0.5411 |
| Associated criterion | >0.5539 |

3.4.4 Logistic regression and ROC curve analysis for the combination of IL 1 beta, IL-2, IL-6 and IL-10

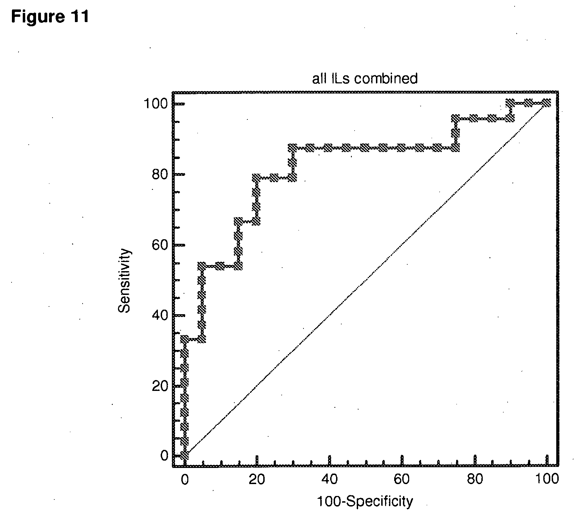

The ability to predict the diagnosis of the patients correctly increased further when the combination of parameters measured for all four interleukins was taken into account and normalised for cell numbers (see Figure 11 and analysis below).

**Logistic regression**

| | |
|---|---|
| Dependent Y | DG |
| Select | |

**OVERALL MODEL FIT**

| | |
|---|---|
| Null model - 2 Log Likelihood | 60.633 |
| Full model -2 Log Likelihood | 46.472 |
| Chi-square | 14.161 |
| DF | 8 |
| Significance level | P = 0.0777 |

**COEFFICIENTS AND STANDARD ERRORS**

| Variable | Coefficient | Std. Error | P |
|---|---|---|---|
| Rapa IL_1_Beta_R/cell | -97833.31096 | 71605.28179 | 0.1718 |
| Control IL_10/cell | -893476.20549 | 499925.93833 | 0.0739 |
| Rapa IL_10/ cell | 1179046.99423 | 675543.69063 | 0.0809 |
| Control IL_2/ cell | 18818.68649 | 13142.67004 | 0.1522 |
| Rapa IL_2/ cell | 27853.09109 | 20494.42955 | 0.1741 |
| Rapa IL_6/ cell | 51427.70147 | 26334.92380 | 0.0508 |
| Change in cell numbers induced by Rapamycin | -0.13229 | 0.091870 | 0.1499 |
| Control cell numbers | -0.45198 | 0.23570 | 0.0552 |
| Constant | 4.2550 | | |

**ROC CURVE ANALYSIS**

| | |
|---|---|
| Area under the ROC curve (AUC) | 0.821 |
| Standard Error | 0.0649 |
| 95% Confidence interval | 0.676 to 0.920 |

**AREA UNDER THE ROC CURVE (AUC)**

| | |
|---|---|
| Area under the ROC curve (AUC) | 0.821 |
| Standard Error[a] | 0.0649 |
| 95% Confidence interval[b] | 0.694 to 0.948 |
| z statistic | 4.944 |
| Significance level P (Area=0.5) | <0.0001 |
| [a] Hanley & McNeil, 1982<br>[b]AUC $\pm$ 1.96 SE | |

**YOUDEN INDEX**

| | |
|---|---|
| Youden index J | 0.5917 |
| Associated criterion | >0.552 |

3.4.5 Logistic regression and ROC curve analysis for the combination of IL 1 beta IL-2, IL-6 and IL-10 with ApoE status

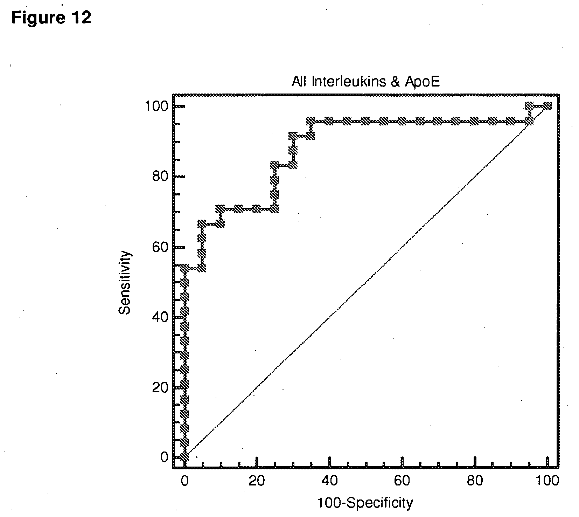

When the ApoE status of the patients is included in the analysis the prediction becomes more accurate (see Figure 12 and analysis below).

**Logistic regression**

| | |
|---|---|
| Dependent Y | DG |
| Select | |

**OVERALL MODEL FIT**

| | |
|---|---|
| Null model - 2 Log Likelihood | 60.633 |
| Full model -2 Log Likelihood | 37.244 |
| Chi-square | 23.389 |
| DF | 2 |
| Significance level | P < 0.0001 |

**COEFFICIENTS AND STANDARD ERRORS**

| Variable | Coefficient | Std. Error | P |
|---|---|---|---|
| all_ILs_combined | 5.69684 | 2.02719 | 0.0050 |
| ApoE4 | 1.98995 | 0.81722 | 0.0149 |
| Constant | -3.7343 | | |

**ROC CURVE ANALYSIS**

| Area under the ROC curve (AUC) | 0.879 |
|---|---|
| Standard Error | 0.0528 |
| 95% Confidence interval | 0.745 to 0.958 |

**ROC curve**

| Variable | All_Interleukins_&_ApoE |
|---|---|
| Classification variable | DG |

**AREA UNDER THE ROC CURVE (AUC)**

| Area under the ROC curve (AUC) | 0.879 |
|---|---|
| Standard Error[a] | 0.0528 |
| 95% Confidence interval[b] | 0.776 to 0.983 |
| z statistic | 7.187 |
| Significance level P (Area=0.5) | <0.0001 |
| [a] Hanley & McNeil, 1982 [b] AUG $\pm$ 1.96 SE | |

**YOUDEN INDEX**

| Youden index J | 0.6167 |
|---|---|
| Associated criterion | >0.3567 |

3.4.6 Effect of rapamycin on cell proliferation and interleukin expression

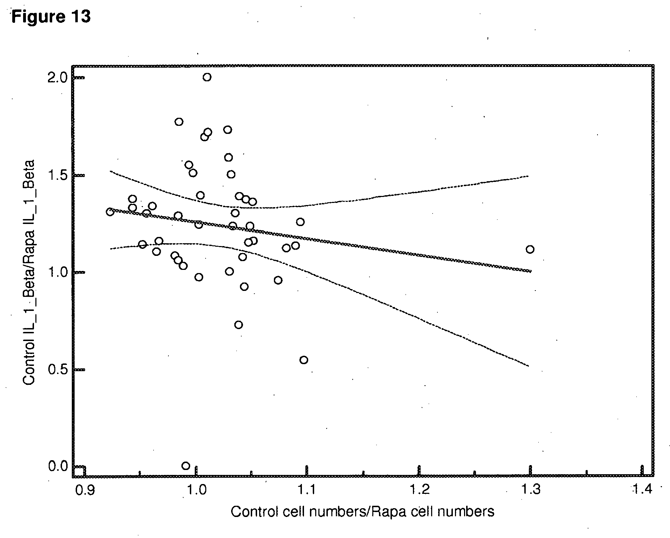

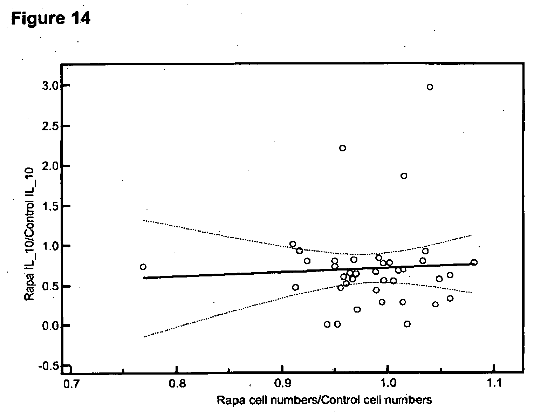

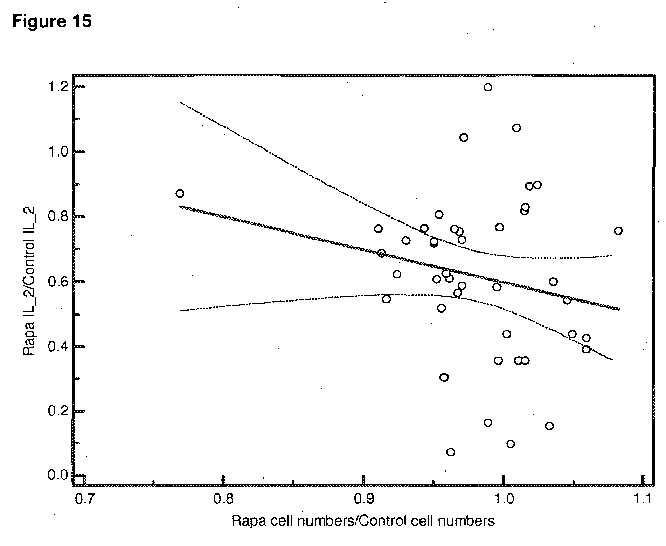

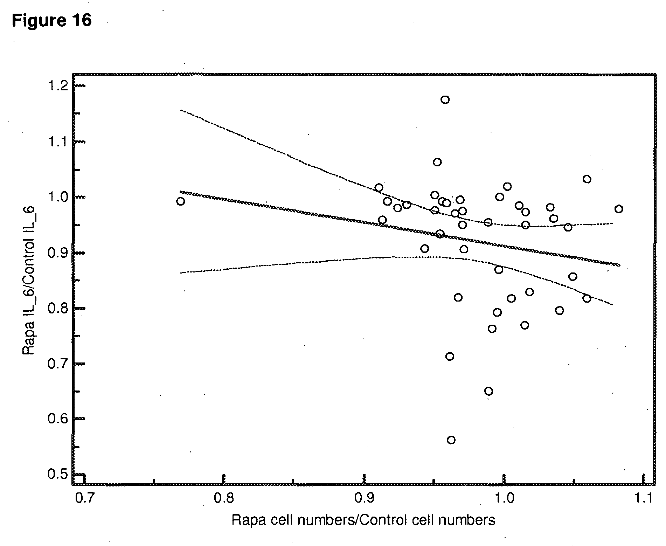

The relationship between changes in interleukin expression and the changes in cell proliferation (actual cell numbers) induced by Rapamycin in these cultures was also analysed. We have found that the changes induced in interleukin levels did not relate significantly to changes induced in cell numbers (Figure 13: IL 1beta, p=0.32, $R^2$=2.37%; Figure 14: IL 10, p=0.73, $R^2$=0.3%; Figure 15: IL 2, p=0.17, $R^2$=4.5%; Figure 16: IL 6, p=0.21, $R^2$=3.7%).

**Claims**

1. A method to assist with diagnosis of Alzheimer's disease in a live human subject, which method comprises screening for a differential response to a cell-division inhibitor in non-neuronal cells taken from the human subject as compared with control non-neuronal cells, wherein the differential response is measured by analysing the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10).

2. A method of assessing the risk of Alzheimer's disease progression in a human subject, which method comprises screening for a differential response to a cell-division inhibitor in non-neuronal cells taken from the human subject as compared with control non-neuronal cells, wherein the differential response is measured by analysing the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10).

3. The method of claim 1 or claim 2 wherein a differential response to a cell-division inhibitor in non-neuronal cells taken from a human subject is determined by:-

(a) inducing cell division in non-neuronal cells taken from the human subject suspected of having Alzheimer's disease;

(b) separating the dividing non-neuronal cells of (a) into two pools and treating one pool of cells with a cell-division inhibitor;

(c) determining the level of expression of one or more of the genes shown in Tables 1 and 2 in the pool of cells treated with the cell-division inhibitor and in the untreated pool of cells;

(d) comparing the level of expression of the one or more genes analysed in (c) for the pool of cells treated with the cell-division inhibitor and the untreated pool of cells, in order to determine any inhibitor-induced relative changes in gene expression;

(e) repeating steps (a)-(d) for control non-neuronal cells;

(f) comparing any inhibitor-induced relative changes in gene expression observed in non-neuronal cells taken from the human subject to be tested with any inhibitor-induced relative changes in gene expression observed in control non-neuronal cells, in order to determine any differences in inhibitor-induced relative gene expression changes between test and control non-neuronal cells, wherein a difference is indicative of Alzheimer's disease.

4. The method of any of claims 1-3 wherein the human subject is asymptomatic for Alzheimer's disease or wherein the human subject exhibits mild cognitive impairment or wherein the human subject exhibits one or more symptoms consistent with Alzheimer's disease.

5. The method of any of the preceding claims wherein the control non-neuronal cells are taken from an age-matched healthy subject and/or wherein the non-neuronal cells are lymphocytes.

6. The method of any of the preceding claims wherein the cell-division inhibitor is a G1 inhibitor or an mTOR inhibitor or rapamycin.

7. The method of any of the preceding claims wherein gene expression is analysed by assessing mRNA levels or wherein gene expression is analysed by assessing levels of the corresponding protein.

8. The method of any one of claims 1-7 wherein the differential response is measured by analysing the relative changes in gene expression of two or more genes wherein a first gene is interleukin 1 beta (IL1B) and at least one other gene is selected from the group consisting of: interleukin 2 (IL-2); interleukin 6 (IL-6); and interleukin 10 (IL-10) or wherein the differential response is measured by analysing the relative changes in gene expression of interleukin 1 beta (IL1B); interleukin 2 (IL-2); interleukin 6 (IL-6); and interleukin 10 (IL-10).

9. The method of any one of the preceding claims wherein the human subject is additionally screened for polymorphisms in the apoE gene, and the results of the apoE genotyping are combined with the results obtained by carrying out the methods of any one of claims 1-7 in order to assist with diagnosis of Alzheimer's disease or assess the risk of Alzheimer's disease progression in the human subject, optionally wherein the subject is screened for the presence of at least one *ApoE4* allele.

10. A method of identifying a compound having potential pharmacological activity in the treatment of Alzheimer's disease, which method comprises screening for a differential response to a cell-division inhibitor in non-neuronal cells, which cells exhibit a cell cycle regulatory defect at the G1/S transition, in the presence and absence of a test compound, wherein the differential response is measured by comparing the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10) with the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10) in control non-neuronal cells, and wherein a compound which results in a reduction in the differential response to the cell-division inhibitor in non-neuronal cells exhibiting a cell cycle regulatory defect at the G1/S transition is identified as having potential pharmacological activity in the treatment of Alzheimer's disease.

11. A method of determining whether a pharmacological agent is likely to be of benefit in the treatment of Alzheimer's disease in a particular human individual, which method comprises screening for a differential response to a cell-division inhibitor in non-neuronal cells taken from the particular human individual, in the presence and absence of a pharmacological agent, wherein the differential response is measured by comparing the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10) with the inhibitor-induced relative changes in gene expression of one or more of the genes selected from the group consisting of: interleukin 1 beta (IL1B);

interleukin 2 (IL-2); interleukin 6 (IL-6); and interleukin 10 (IL-10) in control non-neuronal cells, and wherein a pharmacological agent which results in a reduction in the differential response to the cell-division inhibitor in non-neuronal cells taken from the human individual is identified as likely to be of benefit in the treatment of Alzheimer's disease in said individual.

12. A method of monitoring the efficacy of a pharmacological agent in the treatment of Alzheimer's disease in a particular human individual, which method comprises screening for a differential response to a cell-division inhibitor in non-neuronal cells taken from the particular human individual, in the presence and absence of a pharmacological agent, wherein the differential response is measured by comparing the inhibitor-induced relative changes in gene expression of one or more of the genes-selected from the group consisting of: interleukin 1 beta (IL1B); interleukin 2 (IL-2); interleukin 6 (IL-6); and interleukin 10 (IL-10) with the inhibitor-induced relative changes in gene expression of one or more of the genes selected from the group consisting of: interleukin 1 beta (IL1B); interleukin 2 (IL-2); interleukin 6 (IL-6); and interleukin 10 (IL-10) in control non-neuronal cells, and wherein a pharmacological agent which results in a reduction in the differential response to the cell-division inhibitor in non-neuronal cells taken from the human individual is identified as efficacious in the treatment of Alzheimer's disease in said individual.

13. The method of any one of claims 10-12 wherein the differential response is measured by analysing the relative changes in gene expression of two or more genes wherein a first gene is interleukin 1 beta (IL1B) and at least one other gene is selected from the group consisting of:

interleukin 2 (IL-2); interleukin 6 (IL-6); and interleukin 10 (IL-10) or
wherein the differential response is measured by analysing the relative changes in gene expression of interleukin 1 beta (IL1B); interleukin 2 (IL-2); interleukin 6 (IL-6); and interleukin 10 (IL-10).

14. A method by which to monitor mTOR signalling in a human cell, which method comprises detecting one or more biomarkers of the mTOR signalling pathway, wherein said biomarkers are selected from the group of rapamycin-sensitive genes consisting of: interleukin 1 beta (IL1B); interleukin 2 (IL-2); interleukin 6 (IL-6); and interleukin 10 (IL-10), optionally wherein the human cell is a lymphocyte.

15. The method of claim 14 wherein the human cell is isolated from a human subject suspected of having Alzheimer's disease, optionally wherein the human subject is asymptomatic for Alzheimer's disease, or optionally wherein the human subject exhibits mild cognitive impairment, or optionally wherein the human subject exhibits one or more symptoms consistent with Alzheimer's disease, and wherein monitoring mTOR signalling is used to assist with the diagnosis of Alzheimer's disease in the human subject.

16. Use of one or more rapamycin-sensitive genes as biomarkers of the mTOR signalling pathway in a human cell wherein said genes are selected from the group consisting of:

interleukin 1 beta (IL1B); interleukin 2 (IL-2); interleukin 6 (IL-6); and interleukin 10 (IL-10), optionally wherein the human cell is a lymphocyte and
wherein the human cell is isolated from a human subject suspected of having Alzheimer's disease.

**Patentansprüche**

1. Verfahren zur Unterstützung bei der Diagnose der Alzheimer-Krankheit bei einer lebenden menschlichen Versuchsperson, wobei das Verfahren ein Screening auf eine Differenzialreaktion auf einen Zellteilungshemmstoff in nicht-neuronalen Zellen aufweist, die der menschlichen Versuchsperson entnommen werden, verglichen mit nicht-neuronalen Steuerzellen, wobei die Differenzialreaktion durch Analysieren der durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) ausgewählt sind, gemessen wird.

2. Verfahren zur Beurteilung des Risikos des Krankheitsverlaufs von Alzheimer bei einer menschlichen Versuchsperson, wobei das Verfahren ein Screening auf eine Differenzialreaktion auf einen Zellteilungshemmstoff in nicht-neuronalen Zellen aufweist, die der menschlichen Versuchsperson entnommen werden, verglichen mit nicht-neuronalen Steuerzellen, wobei die Differenzialreaktion durch Analysieren der durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) ausgewählt sind, gemessen wird.

3.  Verfahren nach Anspruch 1 oder Anspruch 2, wobei eine Differenzialreaktion auf einen Zellteilungshemmstoff in nicht-neuronalen Zellen, die von einer menschlichen Versuchsperson entnommen werden, durch Folgendes bestimmt wird:

    (a) Verursachen einer Zellteilung in nicht-neuronalen Zellen, die der menschlichen Versuchsperson entnommen werden, von welcher vermutet wird, dass sie an der Alzheimer-Krankheit leidet;
    (b) Auftrennen des Teilens der nicht-neuronalen Zellen von (a) in zwei Pools und Behandeln eines Pools von Zellen mit einem Zellteilungshemmstoff;
    (c) Bestimmen des Grads der Expression von einem oder mehreren der Gene, die in Tabelle 1 und 2 dargestellt sind, in dem Pool von Zellen, die mit dem Zellteilungshemmstoff behandelt wurden, und in dem unbehandelten Pool von Zellen;
    (d) Vergleichen des Grads der Expression des einen oder der mehreren Gene, die in (c) analysiert werden, für den Pool von Zellen, die mit dem Zellteilungshemmstoff behandelt wurden, und den unbehandelten Pool von Zellen, um alle von dem Hemmstoff verursachten relativen Veränderungen bei der Genexpression zu bestimmen;
    (e) Wiederholen der Schritte (a)-(d) an den nicht-neuronalen Steuerzellen;
    (f) Vergleichen aller von dem Hemmstoff verursachten relativen Veränderungen bei der Genexpression, die bei nicht-neuronalen Zellen beobachtet werden, die der zu testenden menschlichen Versuchsperson entnommen werden, mit allen von dem Hemmstoff verursachten relativen Veränderungen bei der Genexpression, die bei nicht-neuronalen Steuerzellen beobachtet werden, um Unterschiede bei den durch Hemmstoffe verursachten relativen Genexpressionsveränderungen zwischen nicht neuronalen Versuchs- und Steuerzellen zu bestimmen, wobei ein Unterschied auf die Alzheimer-Krankheit hinweist.

4.  Verfahren nach einem der Ansprüche 1 bis 3, wobei die menschliche Versuchsperson asymptomatisch bezüglich der Alzheimer-Krankheit ist oder wobei die menschliche Versuchsperson geringe kognitive Beeinträchtigungen aufweist oder wobei die menschliche Versuchsperson ein Symptom oder mehrere Symptome aufweist, die mit der Alzheimer-Krankheit übereinstimmen.

5.  Verfahren nach einem der vorhergehenden Ansprüche, wobei die nicht-neuronalen Steuerzellen einer gleichaltrigen gesunden Versuchsperson entnommen werden und/oder wobei die nicht-neuronalen Zellen Lymphozyten sind.

6.  Verfahren nach einem der vorhergehenden Ansprüche, wobei der Zellteilungshemmstoff ein G1-Hemmstoff oder ein mTOR-Hemmstoff oder Rapamycin ist.

7.  Verfahren nach einem der vorhergehenden Ansprüche, wobei die Genexpression durch Beurteilen des mRNA-Gehalts analysiert wird oder wobei die Genexpression durch Beurteilen des Gehalts des entsprechenden Proteins analysiert wird.

8.  Verfahren nach einem der Ansprüche 1 bis 7, wobei die Differenzialreaktion durch Analysieren der relativen Veränderungen bei der Genexpression von zwei oder mehreren Genen gemessen wird, wobei ein erstes Gen Interleukin 1 beta (IL1B) ist und wenigstens ein weiteres Gen aus der Gruppe bestehend aus Interleukin 2 (IL-2); Interleukin 6 (IL-6) und Interleukin 10 (IL-10) ausgewählt ist, oder wobei die Differenzialreaktion durch Anlaysieren der relativen Veränderungen bei der Genexpression von Interleukin 1 beta (IL1B); Interleukin 2 (IL-2); Interleukin 6 (IL-6) und Interleukin 10 (IL-10) gemessen wird.

9.  Verfahren nach einem der vorhergehenden Ansprüche, wobei an der menschlichen Versuchsperson zusätzlich ein Screening auf Polymorphismen im apoE-Gen ausgeführt wird, und wobei die Ergebnisse der apoE-Genotypisierung mit den Ergebnissen kombiniert werden, die durch Ausführen der Verfahren nach einem der Ansprüche 1 bis 7 erhalten werden, um bei der Diagnose der Alzheimer-Krankheit zu helfen oder das Risiko des Krankheitsverlaufs von Alzheimer bei der menschlichen Versuchsperson zu beurteilen, wobei optional an der Versuchsperson ein Screening auf das Vorhandensein von wenigstens einem ApoE4-Allel ausgeführt wird.

10. Verfahren zur Identifikation einer Zusammensetzung mit potentieller pharmakologischer Aktivität bei der Behandlung der Alzheimer-Krankheit, wobei das Verfahren ein Screening auf eine Differenzialreaktion auf einen Zellteilungshemmstoff in nicht-neuronalen Zellen aufweist, welche Zellen einen regulatorischen Zellzykius-Defekt am G1/S-Übergang aufweisen, in Gegenwart und in Abwesenheit einer Versuchszusammensetzung, wobei die Differenzialreaktion durch Vergleichen der durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) ausgewählt sind, mit den durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) in nicht-neuronalen Steuerzellen ausgewählt sind, gemessen wird, und wobei eine Zusammensetzung, die zu einer Verringerung der Differenzialreaktion auf den Zellteilungshemmstoff in nicht-neuronalen Zellen führt, die einen regulatorischen Zellzyklus-Defekt am G1/S-Übergang aufweisen, als eine potentielle pharmakologische Aktivität bei der Behandlung der Alzheimer-Krankheit aufweisend identifiziert ist.

11. Verfahren zur Bestimmung, ob ein pharmakologisches Mittel voraussichtlich von Vorteil bei der Behandlung der Alzheimer-Krankheit bei einem bestimmten menschlichen Individuum ist, wobei das Verfahren ein Screening auf eine Differenzialreaktion auf einen Zellteilungshemmstoff in nicht-neuronalen Zellen aufweist, die dem bestimmten menschlichen Individuum entnommen werden, in Gegenwart und in Abwesenheit eines pharmakologischen Mittels, wobei die Differenzialreaktion durch Vergleichen der durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) ausgewählt sind, mit den durch den Hemmstoff verursachten relativen Veränderungen bei der Genexpression von einem oder mehreren der Gene, die aus der Gruppe bestehend aus Interleukin 1 beta (IL1 B); Interleukin 2 (IL-2); Interleukin 6 (IL-6) und Interleukin 10 (IL-10) in nicht-neuronalen Steuerzellen ausgewählt sind, gemessen wird, und wobei ein pharmakologisches Mittel, das zu einer Verringerung der Differenzialreaktion auf den Zellteilungshemmstoff in nicht-neuronalen Zellen führt, die dem menschlichen Individuum entnommen werden, als voraussichtlich von Vorteil bei der Behandlung der Alzheimer-Krankheit bei diesem Individuum identifiziert ist.

12. Verfahren zur Überwachung der Wirksamkeit eines pharmakologischen Mittels bei der Behandlung der Alzheimer-Krankheit bei einem bestimmten menschlichen Individuum, wobei das Verfahren ein Screening auf eine Differenzialreaktion auf einen Zellteilungshemmstoff in nicht-neuronalen Zellen aufweist, die dem bestimmten menschlichen Individuum entnommen werden, in Gegenwart und in Abwesenheit eines pharmakologischen Mittels, wobei die Differenzialreaktion durch Vergleichen der durch den Hemmstoff verursachten relativen Veränderungen bei der Genexpression von einem oder mehreren der Gene, die aus der Gruppe bestehend aus Interleukin 1 beta (IL1B); Interleukin 2 (IL-2); Interleukin 6 (IL-6) und Interleukin 10 (IL-10) ausgewählt sind, mit den durch den Hemmstoff verursachten relativen Veränderungen bei der Genexpression von einem oder mehreren der Gene, die aus der Gruppe bestehend aus Interleukin 1 beta (IL1 B); Interleukin 2 (IL-2); Interleukin 6 (IL-6) und Interleukin 10 (IL-10) in nicht-neuronalen Steuerzellen ausgewählt sind, gemessen wird, und wobei ein pharmakologisches Mittel, das zu einer Verringerung der Differenzialreaktion auf den Zellteilungshemmstoff in nicht-neuronalen Zellen führt, die dem menschlichen Individuum entnommen werden, als wirksam bei der Behandlung der Alzheimer-Krankheit bei diesem Individuum identifiziert ist.

13. Verfahren nach einem der Ansprüche 10 bis 12, wobei die Differenzialreaktion durch Analysieren der relativen Veränderungen bei der Genexpression von zwei oder mehreren Genen gemessen wird, wobei ein erstes Gen Interleukin 1 beta (IL1B) ist und wenigstens ein weiteres Gen aus der Gruppe bestehend aus Interleukin 2 (IL-2); Interleukin 6 (IL-6) und Interleukin 10 (IL-10) ausgewählt ist, oder wobei die Differenzialreaktion durch Anlaysieren der relativen Veränderungen bei der Genexpression von Interleukin 1 beta (IL1B); Interleukin 2 (IL-2); Interleukin 6 (IL-6) und Interleukin 10 (IL-10) gemessen wird.

14. Verfahren, zum Überwachen einer mTOR-Signalisierung in einer menschlichen Zelle, wobei das Verfahren ein Erfassen eines oder mehrerer Biomarker des mTOR-Signalisierungspfads aufweist, wobei die Biomarker aus der Gruppe aus Rapamycin-sensitiven Genen ausgewählt sind, die aus Interleukin 1 beta (IL1 B); Interleukin 2 (IL-2); Interleukin 6 (IL-6) und Interleukin 10 (IL-10) besteht, wobei optional die menschliche Zelle ein Lymphozyt ist.

15. Verfahren nach Anspruch 14, wobei die menschliche Zelle von einer menschlichen Versuchsperson isoliert wird, von welcher vermutet wird, dass sie an der Alzheimer-Krankheit leidet, wobei optional die menschliche Versuchsperson asymptomatisch bezüglich der Alzheimer-Krankheit ist, oder wobei optional die menschliche Versuchsperson geringe kognitive Beeinträchtigungen aufweist, oder wobei optional die menschliche Versuchsperson ein Symptom oder mehrere Symptome aufweist, die mit der Alzheimer-Krankheit übereinstimmen, und wobei die Überwachung der mTOR-Signalisierung dazu verwendet wird, bei der Diagnose der Alzheimer-Krankheit in der menschlichen Versuchsperson zu helfen.

**16.** Verwendung von einem oder mehreren Rapamycin-sensitiven Genen als Biomarker des mTOR-Signalisierungspfads in einer menschlichen Zelle, wobei die Gene aus der Gruppe bestehend aus Interleukin 1 beta (IL1 B); Interleukin 2 (IL-2); Interleukin 6 (IL-6) und Interleukin 10 (IL-10) ausgewählt sind, wobei optional die menschliche Zelle ein Lymphozyt ist und wobei die menschliche Zelle von einer menschlichen Versuchsperson isoliert wird, von welcher vermutet wird, dass sie an der Alzheimer-Krankheit leidet.

**Revendications**

1. Procédé pour faciliter le diagnostic de la maladie d'Alzheimer chez un sujet humain vivant, le procédé comprenant le criblage d'une réponse différentielle à un inhibiteur de la division cellulaire dans des cellules non neuronales prélevées chez le sujet humain par comparaison à des cellules non neuronales de contrôle, où la réponse différentielle est mesurée en analysant les modifications relatives de l'expression génique, induites par l'inhibiteur, d'un ou de plusieurs des gènes sélectionnés dans le groupe consistant en : l'interleukine 1 bêta (IL1B) ; l'interleukine 2 (IL-2) ; l'interleukine 6 (IL-6) ; et l'interleukine 10 (IL-10).

2. Procédé pour évaluer le risque de progression de la maladie d'Alzheimer chez un sujet humain, le procédé comprenant le criblage d'une réponse différentielle à un inhibiteur de la division cellulaire dans des cellules non neuronales prélevées chez le sujet humain par comparaison à des cellules non neuronales de contrôle, où la réponse différentielle est mesurée en analysant les modifications relatives de l'expression génique, induites par l'inhibiteur, d'un ou de plusieurs des gènes sélectionnés dans le groupe consistant en : l'interleukine 1 bêta (IL1B) ; l'interleukine 2 (IL-2) ; l'interleukine 6 (IL-6) ; et l'interleukine 10 (IL-10).

3. Procédé selon la revendication 1 ou la revendication 2, dans lequel une réponse différentielle à un inhibiteur de la division cellulaire dans des cellules non neuronales prélevées chez un sujet humain est déterminée par :

   (a) l'induction de la division cellulaire dans des cellules non neuronales prélevées chez le sujet humain suspecté de présenter la maladie d'Alzheimer ;
   (b) la séparation des cellules non neuronales en division de (a) en deux pools et le traitement d'un pool de cellules avec un inhibiteur de la division cellulaire ;
   (c) la détermination du taux d'expression d'un ou de plusieurs des gènes montrés dans les Tableaux 1 et 2 dans le pool de cellules traité avec l'inhibiteur de la division cellulaire et dans le pool de cellules non traité ;
   (d) la comparaison du taux d'expression du un ou plusieurs gènes analysés en (c) pour le pool de cellules traité avec l'inhibiteur de la division cellulaire et le pool de cellules non traité, afin de déterminer de quelconques modifications relatives de l'expression génique induites par l'inhibiteur ;
   (e) la répétition des étapes (a) - (d) pour des cellules non neuronales de contrôle ;
   (f) la comparaison de quelconques modifications relatives de l'expression génique induites par l'inhibiteur observées dans les cellules non neuronales prélevées chez le sujet humain devant être testées avec de quelconques modifications relatives de l'expression génique induites par l'inhibiteur observées dans les cellules non neuronales de contrôle, afin de déterminer de quelconques différences de modifications relatives de l'expression génique induites par l'inhibiteur entre les cellules non neuronales de test et de contrôle, où une différence indique la maladie d'Alzheimer.

4. Procédé selon l'une quelconque des revendications 1 à 3, dans lequel le sujet humain est asymptomatique pour la maladie d'Alzheimer ou dans lequel le sujet humain exhibe un déficit cognitif léger ou dans lequel le sujet humain exhibe un ou plusieurs symptômes évoquant la maladie d'Alzheimer.

5. Procédé selon l'une quelconque des revendications précédentes, dans lequel les cellules non neuronales de contrôle sont prélevées chez un sujet en bonne santé de même âge et/ou dans lequel les cellules non neuronales sont des lymphocytes.

6. Procédé selon l'une quelconque des revendications précédentes, dans lequel l'inhibiteur de la division cellulaire est un inhibiteur de G1 ou un inhibiteur de mTOR ou la rapamycine.

7. Procédé selon l'une quelconque des revendications précédentes, dans lequel l'expression génique est analysée en évaluant les taux d'ARNm ou dans lequel l'expression génique est analysée en évaluant les taux de la protéine correspondante.

8. Procédé selon l'une quelconque des revendications 1 à 7, dans lequel la réponse différentielle est mesurée en analysant les modifications relatives de l'expression génique de deux gènes ou plus, où un premier gène est l'interleukine 1 bêta (IL1B) et au moins un autre gène est sélectionné dans le groupe consistant en : l'interleukine 2 (IL-2) ; l'interleukine 6 (IL-6) ; et l'interleukine 10 (IL-10) ou dans lequel la réponse différentielle est mesurée en analysant les modifications relatives de l'expression génique de l'interleukine 1 bêta (IL1B) ; l'interleukine 2 (IL-2) ; l'interleukine 6 (IL-6) ; et l'interleukine 10 (IL-10).

9. Procédé selon l'une quelconque des revendications précédentes, dans lequel le sujet humain est en outre criblé pour des polymorphismes dans le gène apoE, et les résultats du génotypage de l'apoE sont combinés avec les résultats obtenus en mettant en oeuvre les procédés selon l'une quelconque des revendications 1 à 7 afin de faciliter le diagnostic de la maladie d'Alzheimer ou d'évaluer le risque de progression de la maladie d'Alzheimer chez le sujet humain, facultativement où le sujet est criblé pour la présente d'au moins un allèle *ApoE4.*

10. Procédé pour identifier un composé possédant une activité pharmacologique potentielle dans le traitement de la maladie d'Alzheimer, le procédé comprenant le criblage d'une réponse différentielle à un inhibiteur de la division cellulaire dans des cellules non neuronales, les cellules exhibant une anomalie de régulation du cycle cellulaire lors de la transition G1/S, en présence et en l'absence d'un composé de test, où la réponse différentielle est mesurée en comparant les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) avec les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) dans des cellules non neuronales de contrôle, et où un composé qui entraîne une réduction de la réponse différentielle à l'inhibiteur de la division cellulaire dans des cellules non neuronales exhibant une anomalie de régulation du cycle cellulaire lors de la transition G1/S est identifié comme possédant une activité pharmacologique potentielle dans le traitement de la maladie d'Alzheimer.

11. Procédé pour déterminer si un agent pharmacologique est susceptible d'être bénéfique dans le traitement de la maladie d'Alzheimer chez un individu humain particulier, le procédé comprenant le criblage d'une réponse différentielle à un inhibiteur de la division cellulaire dans des cellules non neuronales prélevées chez l'individu humain particulier, en présence et en l'absence d'un agent pharmacologique, où la réponse différentielle est mesurée en comparant les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) avec les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) dans des cellules non neuronales de contrôle, et où un agent pharmacologique qui entraîne une réduction de la réponse différentielle à l'inhibiteur de la division cellulaire dans des cellules non neuronales prélevées chez l'individu humain est identifié comme étant susceptible d'être bénéfique dans le traitement de la maladie d'Alzheimer chez ledit individu.

12. Procédé pour surveiller l'efficacité d'un agent pharmacologique dans le traitement de la maladie d'Alzheimer chez un individu humain particulier, le procédé comprenant le criblage d'une réponse différentielle à un inhibiteur de la division cellulaire dans des cellules non neuronales prélevées chez l'individu humain particulier, en présence et en l'absence d'un agent pharmacologique, où la réponse différentielle est mesurée en comparant les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) avec les modifications relatives de l'expression génique, induites par l'inhibiteur, de un ou plusieurs des gènes sélectionnés dans le groupe consistant en : l'interleukine 1 bêta (IL1B); l'interleukine 2 (IL-2) ; l'interleukine 6 (IL-6) ; et l'interleukine 10 (IL-10) dans des cellules non neuronales de contrôle, et où un agent pharmacologique qui entraîne une réduction de la réponse différentielle à l'inhibiteur de la division cellulaire dans des cellules non neuronales prélevées chez l'individu humain est identifié comme étant efficace dans le traitement de la maladie d'Alzheimer chez ledit individu.

13. Procédé selon l'une quelconque des revendications 10 à 12, dans lequel la réponse différentielle est mesurée en analysant les modifications relatives de l'expression génique de deux gènes ou plus, où un premier gène est l'interleukine 1 bêta (IL1B) et au moins un autre gène est sélectionné dans le groupe consistant en : l'interleukine 2 (IL-2) ; l'interleukine 6 (IL-6) ; et l'interleukine 10 (IL-10) ou dans lequel la réponse différentielle est mesurée en analysant les modifications relatives de l'expression génique de l'interleukine 1 bêta (IL1B); l'interleukine 2 (IL-2) ; l'interleukine 6 (IL-6) ; et l'interleukine 10 (IL-10).

14. Procédé permettant de surveiller la signalisation de mTOR dans une cellule humaine, le procédé comprenant la détection d'un ou de plusieurs biomarqueurs de la voie de signalisation de mTOR, où lesdits biomarqueurs sont sélectionnés dans le groupe des gènes sensibles à la rapamycine consistant en : l'interleukine 1 bêta (IL1B); l'interleukine 2 (IL-2) ; l'interleukine 6 (IL-6) ; et l'interleukine 10 (IL-10), facultativement où la cellule humaine est un lymphocyte.

15. Procédé selon la revendication 14, dans lequel la cellule humaine est isolée à partir d'un sujet humain suspecté de présenter la maladie d'Alzheimer, facultativement dans lequel le sujet humain est asymptomatique pour la maladie d'Alzheimer, ou facultativement dans lequel le sujet humain exhibe un déficit cognitif léger, ou facultativement dans lequel le sujet humain exhibe un ou plusieurs symptômes évoquant la maladie d'Alzheimer, et dans lequel la surveillance de la signalisation de mTOR est utilisée pour faciliter le diagnostic de la maladie d'Alzheimer chez le sujet humain.

16. Utilisation d'un ou de plusieurs gène sensibles à la rapamycine en tant que biomarqueurs de la voie de signalisation de mTOR dans une cellule humaine, où lesdits gènes sont sélectionnés dans le groupe consistant en : l'interleukine 1 bêta (IL1B); l'interleukine 2 (IL-2) ; l'interleukine 6 (IL-6) ; et l'interleukine 10 (IL-10), facultativement où la cellule humaine est un lymphocyte, et où la cellule humaine est isolée à partir d'un sujet humain suspecté de présenter la maladie d'Alzheimer.

**Figure 1**

**Figure 2**

**Figure 3**

**Figure 4**

**Figure 5**

Figure 6

Figure 7

**Figure 8**

**Figure 9**

IL 1 beta pred

**Figure 10**

IL1 beta & IL 6 pred

**Figure 11**

**Figure 12**

All Interleukins & ApoE

**Figure 13**

Figure 14

**Figure 15**

**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02073212 A
- WO 2011007155 A

### Non-patent literature cited in the description

- **MCKHANN, G. et al.** *Neurology,* 1984, vol. 34, 939-944
- **FORLENZA et al.** *BMC Medicine,* 2010, vol. 8, 89
- **SPERLING et al.** *Criteria for preclinical Alzheimer's disease,* 2010, www.alz.org/research/diagnostic_criteria/preclinical_recommendations.pdf
- **GUSTAW-ROTHENBERG et al.** *Biomark. Med.,* 2010, vol. 4 (1), 15-26
- **NAGY.** *J. Cell. Mol. Med,* 2005, vol. 9 (3), 531-541
- **WINBLAD et al.** *J. Intern. Med,* 2004, vol. 256, 240-246
- **WARING SC ; ROSENBERG RN.** Genome-Wide Association Studies in Alzheimer Disease. *Arch Neurol,* 2008, vol. 65 (3), 329-334
- **CEDAZO-MINGUEZ A.** Apolipoprotein E and Alzheimer's disease: molecular mechanisms and therapeutic opportunities. *J. Cell. Mol. Med.,* 2007, vol. 11 (6), 1227-1238